# EUROPEAN PATENT SPECIFICATION

(11) **EP 3 957 367 B1**
(45) Date of publication and mention of the grant of the patent: **14.01.2026**
(21) Application number: 20766492.1
(22) Date of filing: 05.03.2020
(51) Int. Cl.: A61K 35/741, A61P 31/04

(54) **ANTIBACTERIAL COMPOSITION AGAINST DRUG-RESISTANT BACTERIA OR PRO-INFLAMMATORY BACTERIA**
ANTIBAKTERIELLE ZUSAMMENSETZUNG GEGEN MEDIKAMENTENRESISTENTE BAKTERIEN ODER PROINFLAMMATORISCHE BAKTERIEN
COMPOSITION ANTIBACTÉRIENNE CONTRE DES BACTÉRIES RÉSISTANTES AUX MÉDICAMENTS OU DES BACTÉRIES PRO-INFLAMMATOIRES

(30) Priority: 07.03.2019 US 201962815101 P
(43) Date of publication of application: 23.02.2022
(73) Proprietor: Keio University, Tokyo, 108-8345 (JP)
(72) Inventor: HONDA Kenya, Tokyo 160-8582 (JP); YASUMA Keiko, Tokyo 160-8582 (JP); ATARASHI Koji, Tokyo 160-8582 (JP); NARUSHIMA Seiko, Tokyo 160-8582 (JP); FURUICHI Munehiro, Tokyo 160-8582 (JP); KAWAGUCHI Takaaki, Tokyo 160-8582 (JP)
(74) Representative: J A Kemp LLP
(86) International application number: PCT/JP2020/009423
(87) International publication number: WO 2020/179868

(56) References cited:
- WO-A1-2019/017389
- WO-A1-2019/017389
- WO-A2-2019/118515

## Description

### [Technical Field]

The present invention relates to an antibacterial composition against drug-resistant bacteria or pro-inflammatory bacteria. The present invention also relates to a pharmaceutical composition or its use for treating, ameliorating, or preventing diseases caused by drug-resistant bacteria or pro-inflammatory bacteria.

### [Background Art]

Various commensal bacteria are resident on mucous membranes such as the digestive tract and the oral cavity, forming a flora as a whole. The resident flora plays a very important role in the physiology and health maintenance of the host. A compositional abnormality of the resident flora is called dysbiosis, and has been gradually revealed to be a cause of various diseases. The advancement of the elucidation of the resident mucosal flora may have a high possibility of developing new disease countermeasures and treatments for various diseases, but the detailed mechanism of such flora has not been sufficiently clarified due to its complexity.

Humans produce and swallow about 1.5 liters of saliva every day. Normally, bacteria contained in saliva (oral bacteria) simply pass through the intestinal tract and do not colonize. However, in some situations, oral bacteria may colonize in the intestinal tract. Particularly in Crohn's disease, liver cirrhosis, and colorectal cancer, it has been reported that colonization of oral bacteria in the intestinal tract was observed even at the early stages of disease onset. In addition, it is known that the colonized oral bacteria affect the pathological conditions of the diseases (NPLs 1 to 6).

Meanwhile, by allowing oral bacteria of patients with Crohn's disease and the like to colonize in the intestinal tract to induce Th1 cells, the present inventors succeeded in isolating, culturing, and identifying the bacteria involved in the onset of the diseases (PTL 1). More specifically, the present inventors found that as a result of oral administration of saliva derived from a patient with Crohn's disease to germ-free mice, interferon gamma (IFN-γ) -producing CD4-positive T-cells (Th1 cells) significantly increased in the large intestine. Then, the present inventors succeeded in isolating and culturing the Kp2H7 strain, which is considered to belong to Klebsiella pneumoniae, from the intestines of the mice in which this increase in Th1 cells was observed. Furthermore, it was also clarified that the bacteria derived from the saliva of patients with Crohn's disease were involved in the development of enteritis by colonizing in the intestinal tract and inducing the proliferation or activation of Th1 cells.

Moreover, the present inventors found that when Kp2H7 strains were orally administered to SPF (specific-pathogen-free) mice, intestinal colonization of these bacterial strains was not observed, unlike the case of the germ-free mice. Furthermore, it was also clarified that by administering an antibiotic to the SPF mice, these bacterial strains might be able to colonize in the intestinal tracts of the mice. Then, from these results, the present inventors considered that the intestinal tract contains intestinal bacteria that inhibit the intestinal colonization of Th1 cell-inducible bacteria such as the Kp2H7 strain, and administration of the antibiotic eliminates the intestinal bacteria from the intestinal tract, making the intestinal colonization of the bacteria possible.

Therefore, the present inventors tried to identify the bacteria that suppress the intestinal colonization of Th1 cell-inducible bacteria among human intestinal bacteria. As a result, the present inventors succeeded in isolating and culturing 68 strains, 37 strains, and 42 strains of intestinal bacterial strains from fecal samples derived from 3 healthy subjects (#K, #F, and #I), respectively, and in determining the sequence of 16S rDNA of each bacterial strain. Furthermore, it was clarified that administration of these bacterial strains suppressed intestinal colonization of Th1 cell-inducible bacteria (PTL 2).

### [Citation List]

### [Patent Literature]

[PTL 1] International Publication No. WO2018/084172
[PTL 2] International Publication No. WO2019/017389

### [Non Patent Literature]

[NPL 1] Y. Chenet et al., Scientific reports 6, 34055 (2016)
[NPL 2] D. Gevers et al., Cell host & microbe 15, 382-392 (2014)
[NPL 3] C. A. Lozupone et al., Cell host & microbe 14, 329-339 (2013)
[NPL 4] I. Vujkovic-Cvijin et al., Science translational medicine 5, 193ra191 (2013)
[NPL 5] N. Qin et al., Nature 513, 59-64 (2014)
[NPL 6] C. L. Sears, W. S. Garrett, Cell host & microbe 15, 317-328 (2014)

### [Summary of Invention]

### [Technical Problem]

The present invention has an object to find an intestinal bacterium having antibacterial activity against drug-resistant bacteria or pro-inflammatory bacteria, and to provide an antibacterial composition against drug-resistant bacteria or pro-inflammatory bacteria containing the intestinal bacterium as an active ingredient as well as a pharmaceutical composition or method for treating, ameliorating, or preventing diseases caused by drug-resistant bacteria or pro-inflammatory bacteria.

### [Solution to Problem]

The present inventors have made earnest studies to achieve the above object, and have clarified as a result that the above-mentioned bacteria that suppress the intestinal colonization of Th1 cell-inducible bacteria (68 strains of intestinal bacteria derived from healthy subject #K, 37 strains of intestinal bacteria derived from healthy subject #F, and 42 strains of intestinal bacteria derived from healthy subject #I) can suppress the intestinal colonization of multidrug-resistant bacteria (carbapenem-resistant Enterobacteriaceae, vancomycin-resistant enterococci, Clostridium difficile, Campylobacter jejuni) and pro-inflammatory bacteria (adherent-invasive Escherichia coli).

Furthermore, regarding the ability to suppress bacterial colonization in the intestine, the present inventors have succeeded in selecting 18 strains from the 37 strains of intestinal bacteria derived from healthy subject #F, which are capable of exerting the same level of suppression ability as the 37 strains. Thus, the present invention has been completed.

Specifically, the present invention is defined in the claims.

### [Advantageous Effects of Invention]

According to the present invention, by suppressing the colonization and the like of drug-resistant bacteria or pro-inflammatory bacteria in the intestinal tract, it is possible to suppress the proliferation or activation of these bacteria, and it is possible to treat, ameliorate, or prevent diseases caused by these bacteria.

### [Brief Description of Drawings]

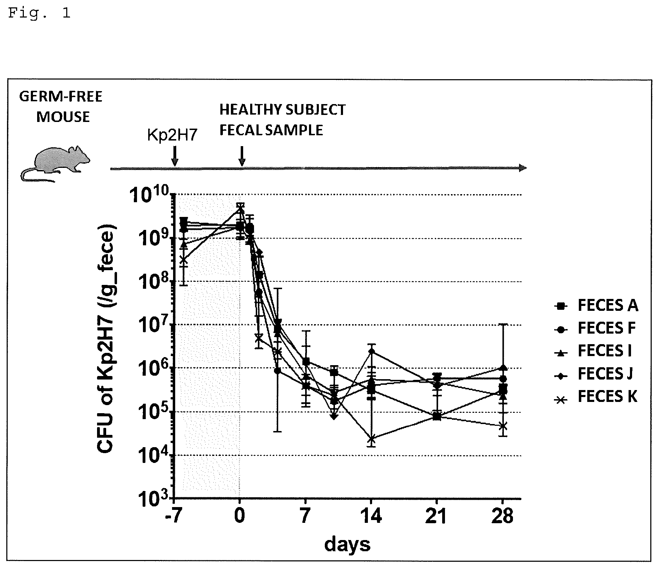

[Fig. 1] Fig. 1 is a graph in CFU showing changes over time in the bacterial amount of fecal Klebsiella when Klebsiella 2H7 strains (Kp2H7) were administered to germ-free mice, and one week later, fecal samples of healthy subjects were administered to the mice (FMT). Five types of feces were used, and Klebsiella was significantly reduced in all samples.

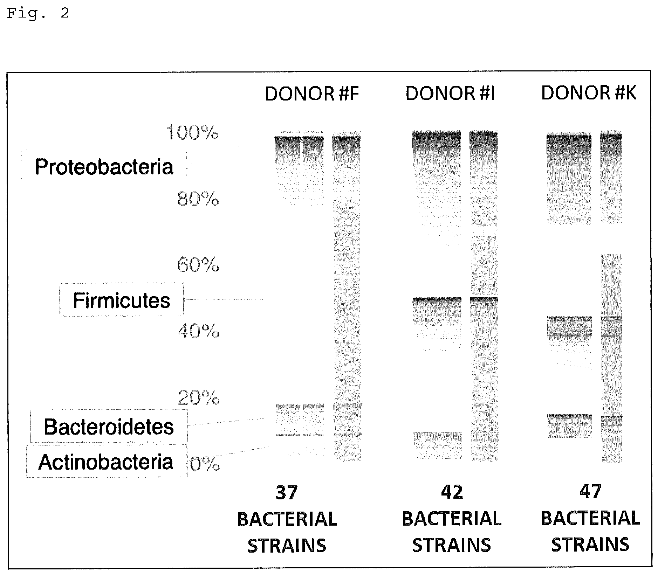

[Fig. 2] Fig. 2 is a bar graph showing the results of 16S meta-analysis of three types of feces derived from healthy donors F, I, and K. Each segment indicates one bacterial strain, and its size indicates the proportion of that bacterium to the total bacterial amount. The three types of feces were cultured in an anaerobic environment, and the strains that could be cultured and isolated there are shown in yellow in the adjacent graph (under color display). The total number of bacteria that could be isolated is shown below.

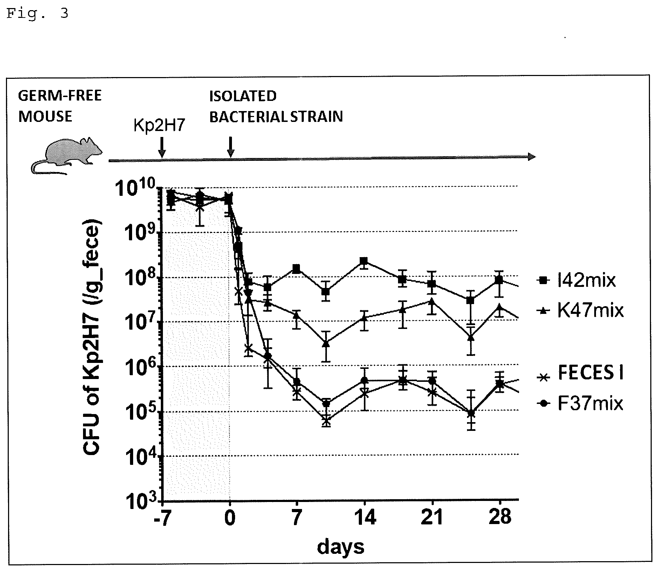

[Fig. 3] Fig. 3 is a graph in CFU showing changes over time in the bacterial amount of fecal Kp2H7 when Kp2H7 was colonized in germ-free mice and then the bacterial strains isolated from feces were mixed and administered. The 37 strains derived from the feces derived from healthy donor F (feces F) reduced Klebsiella as much as the fecal sample.

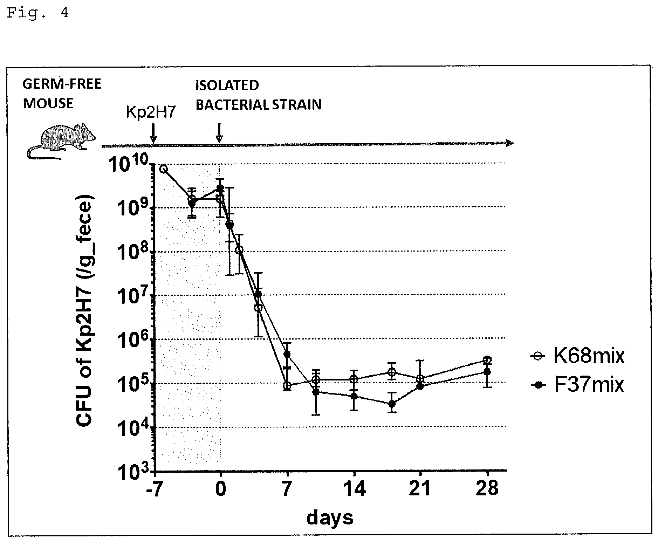

[Fig. 4] Fig. 4 is a graph in CFU showing changes over time in the bacterial amount of fecal Kp2H7 when Kp2H7 was colonized in germ-free mice and then the bacterial strains isolated from feces were mixed and administered. The 37 strains derived from feces F and 68 bacterial strains isolated from the feces derived from healthy donor K (feces K) reduced Klebsiella as much as the fecal sample.

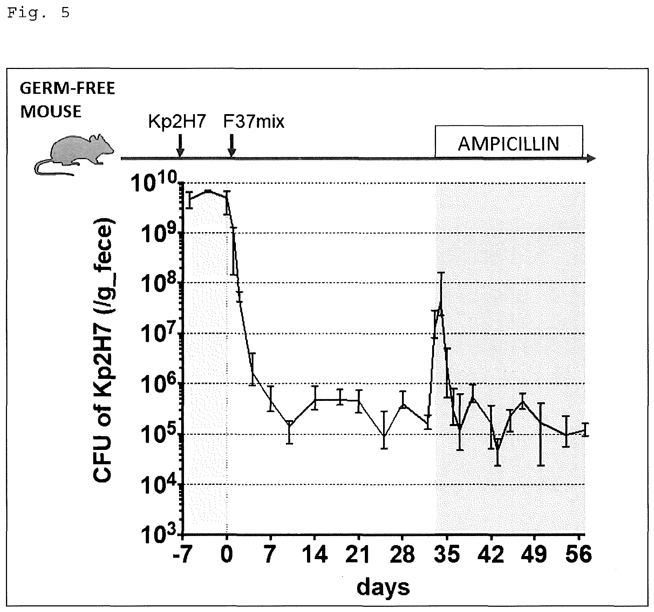

[Fig. 5] Fig. 5 is a graph in CFU showing changes over time in the bacterial amount of fecal Kp2H7 when Kp2H7 was administered to germ-free mice, then F37mix (37 bacterial strains derived from feces F) was administered one week later, and ampicillin was further administered by drinking water one month later. Klebsiella transiently increased with ampicillin administration, but then decreased again.

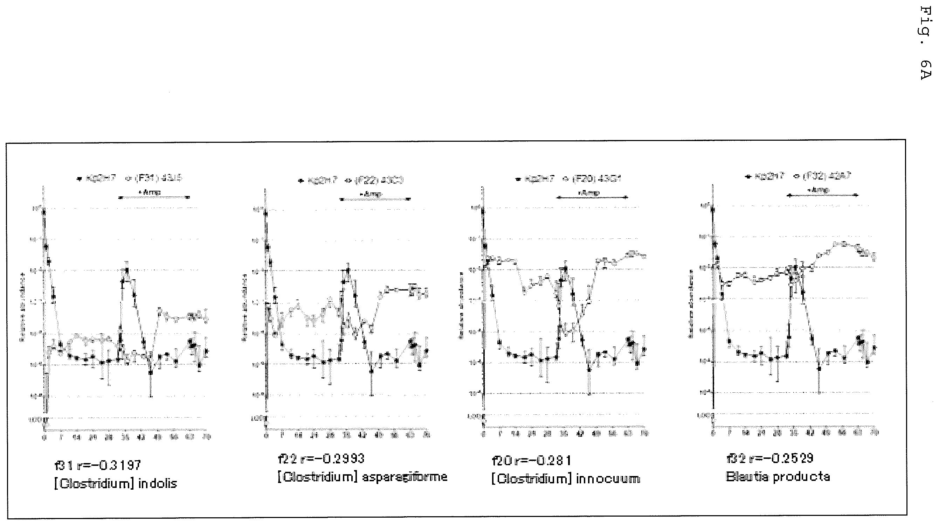

[Fig. 6A] Fig. 6A is a diagram showing changes over time in the abundance ratio of each bacterial amount (F31, F22, F20, F32) in the total bacterial amount in the experiment shown in Fig. 5. Below the figure, the number of each bacterium, r (Spearman's rank correlation coefficient with Klebsiella), and the bacterium's name are shown.

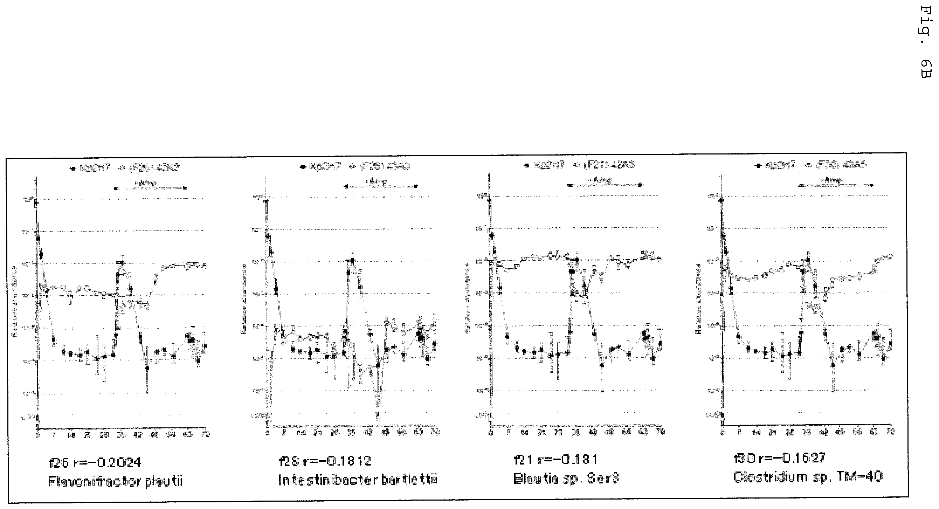

[Fig. 6B] Fig. 6B is a diagram showing changes over time in the abundance ratio of each bacterial amount (F26, F28, F21, F30) in the total bacterial amount in the experiment shown in Fig. 5. Below the figure, the number of each bacterium, r (Spearman's rank correlation coefficient with Klebsiella), and the bacterium's name are shown.

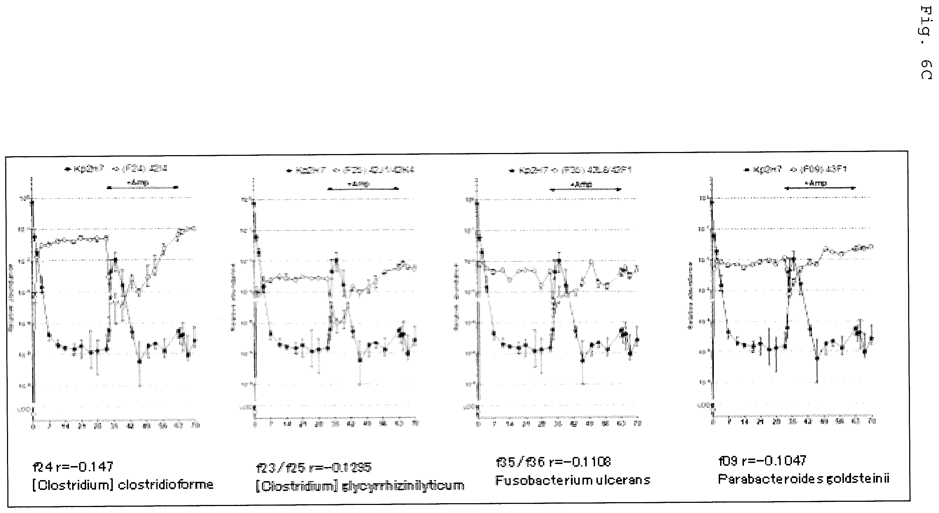

[Fig. 6C] Fig. 6C is a diagram showing changes over time in the abundance ratio of each bacterial amount (F24, F23/F25, F35/F36, F09) in the total bacterial amount in the experiment shown in Fig. 5. Below the figure, the number of each bacterium, r (Spearman's rank correlation coefficient with Klebsiella), and the bacterium's name are shown.

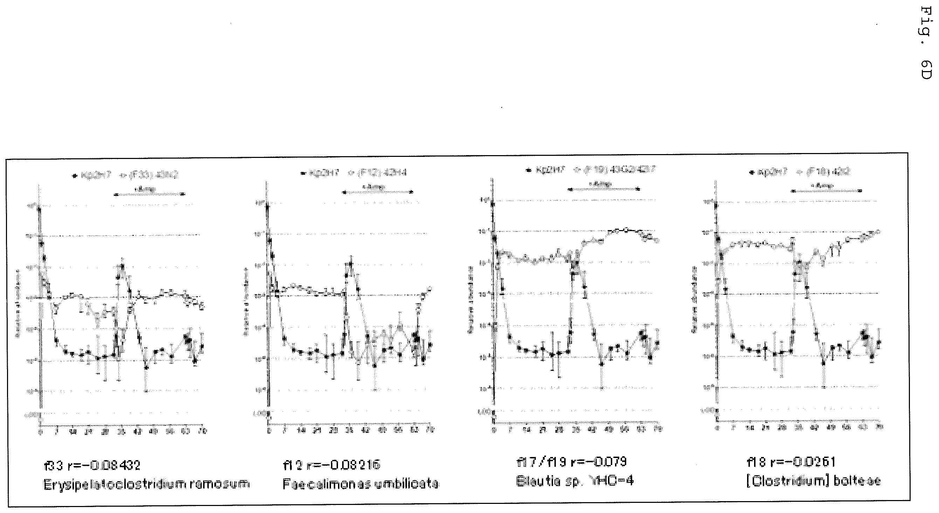

[Fig. 6D] Fig. 6D is a diagram showing changes over time in the abundance ratio of each bacterial amount (F33, F12, F17/F19, F18) in the total bacterial amount in the experiment shown in Fig. 5. Below the figure, the number of each bacterium, r (Spearman's rank correlation coefficient with Klebsiella), and the bacterium's name are shown.

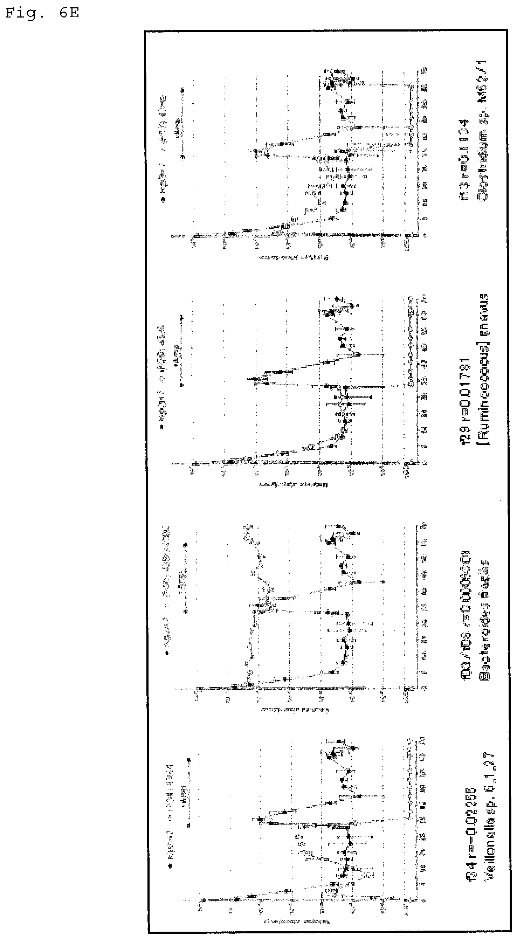

[Fig. 6E] Fig. 6E is a diagram showing changes over time in the abundance ratio of each bacterial amount (F34, F03/F08, F29, F13) in the total bacterial amount in the experiment shown in Fig. 5. Below the figure, the number of each bacterium, r (Spearman's rank correlation coefficient with Klebsiella), and the bacterium's name are shown.

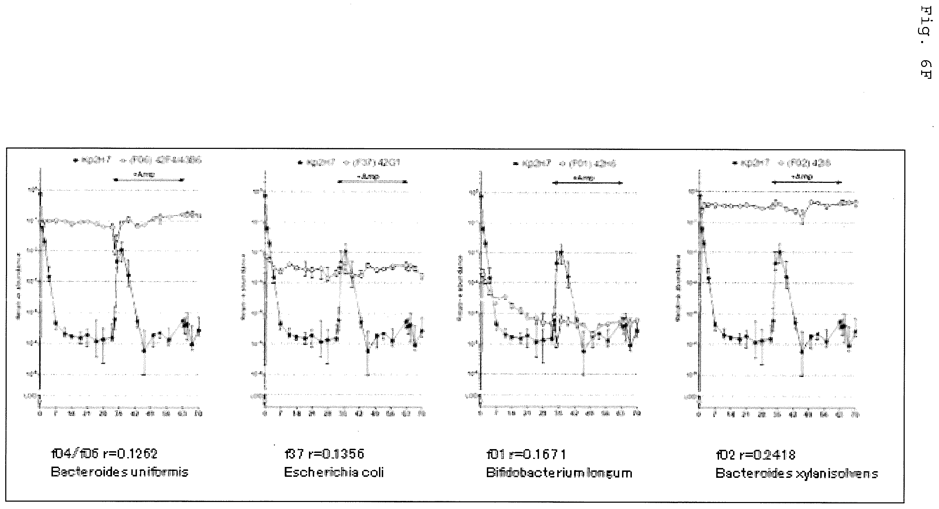

[Fig. 6F] Fig. 6F is a diagram showing changes over time in the abundance ratio of each bacterial amount (F04/F08, F37, F01, F02) in the total bacterial amount in the experiment shown in Fig. 5. Below the figure, the number of each bacterium, r (Spearman's rank correlation coefficient with Klebsiella), and the bacterium's name are shown.

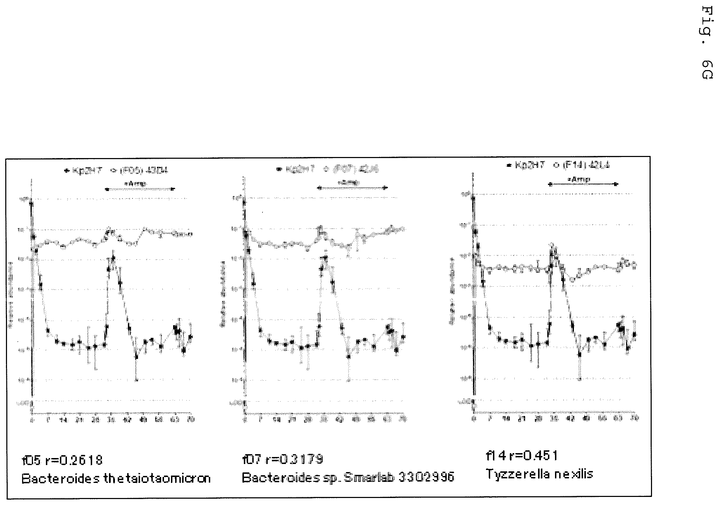

[Fig. 6G] Fig. 6G is a diagram showing changes over time in the abundance ratio of each bacterial amount (F05, F07, F14) in the total bacterial amount in the experiment shown in Fig. 5. Below the figure, the number of each bacterium, r (Spearman's rank correlation coefficient with Klebsiella), and the bacterium's name are shown.

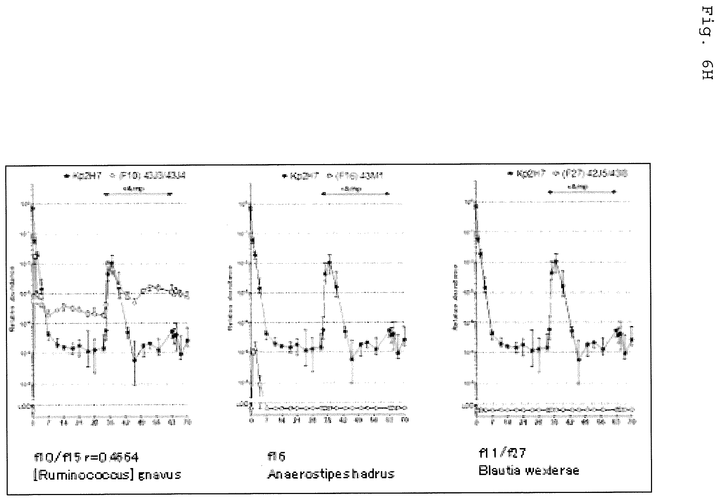

[Fig. 6H] Fig. 6H is a diagram showing changes over time in the abundance ratio of each bacterial amount (F10/F15, F16, F11/F27) in the total bacterial amount in the experiment shown in Fig. 5. Below the figure, the number of each bacterium, r (Spearman's rank correlation coefficient with Klebsiella), and the bacterium's name are shown.

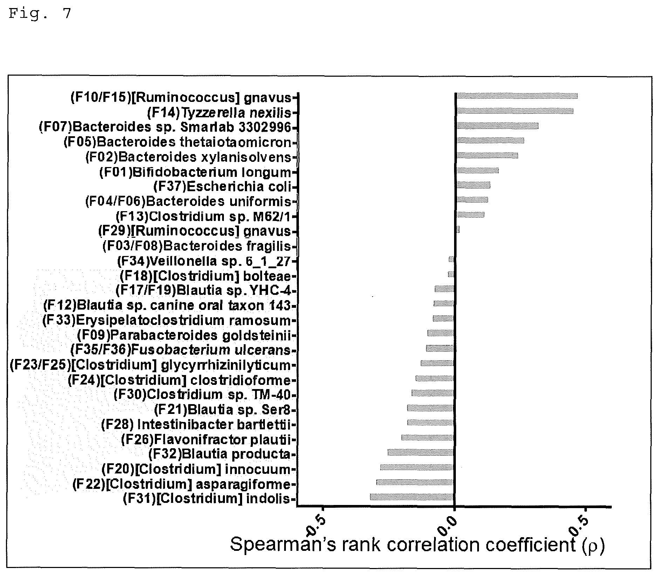

[Fig. 7] Fig. 7 is a diagram in which bacteria are arranged in the order of positive correlation in Spearman's rank correlation coefficient between Kp2H7 and each bacterium in terms of bacterial amount. Many of the Bacteroides are not related in movement to Kp2H7, and many of the negatively correlated ones are in the Firmicutes genus.

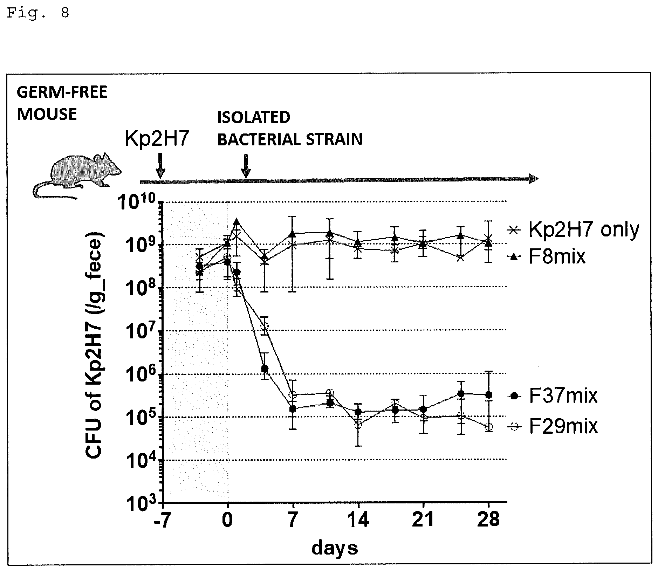

[Fig. 8] Fig. 8 is a graph in CFU showing changes over time in the bacterial amount of fecal Kp2H7 when Kp2H7 was administered to germ-free mice for colonization, and then the 37 strains shown in Fig. 7 (F37mix), 8 strains of the 37 strains belonging to the Bacteroidetes genus (F8mix), or the 29 other bacterial strains (F29mix) were mixed and administered. The 29 strains excluding Bacteroides also showed a decrease in Klebsiella that was almost the same as the 37 strains, and it is considered that Bacteroides is unnecessary for the elimination of Klebsiella.

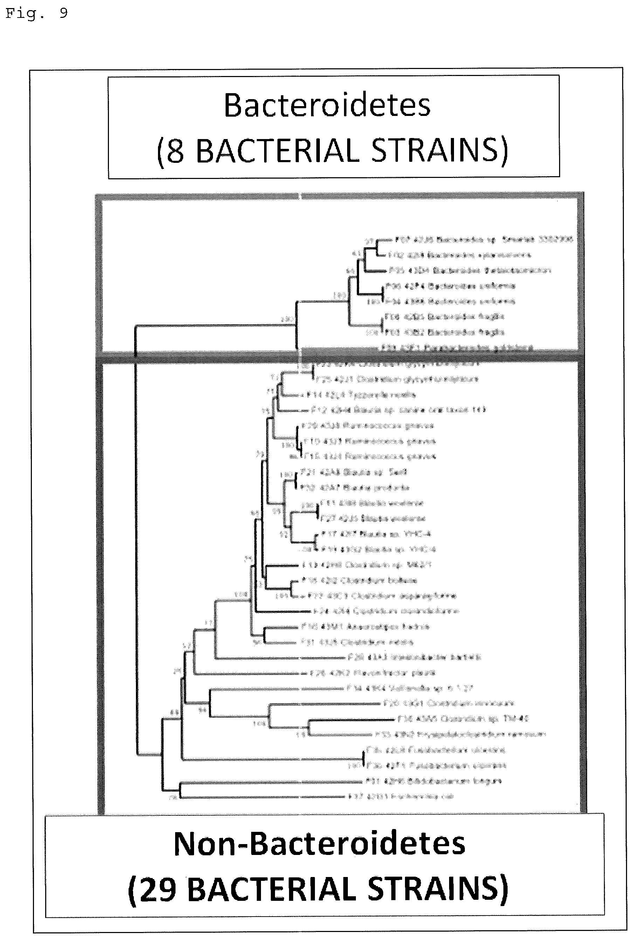

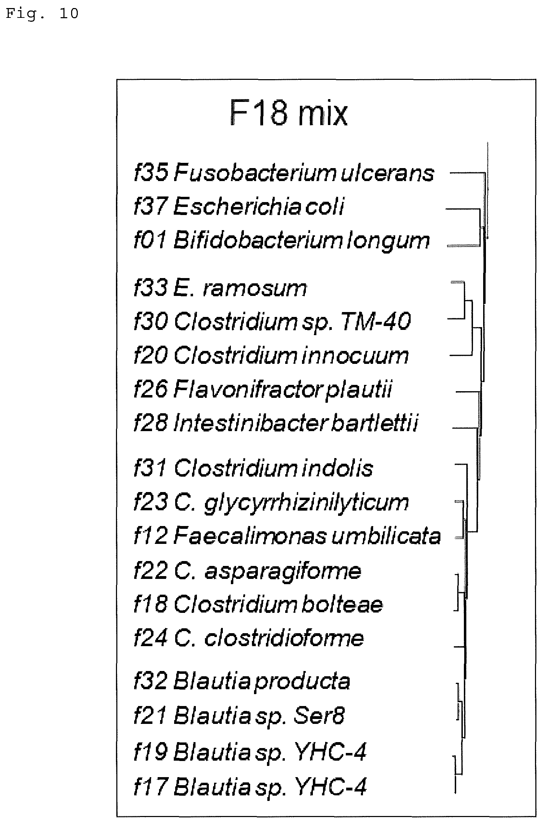

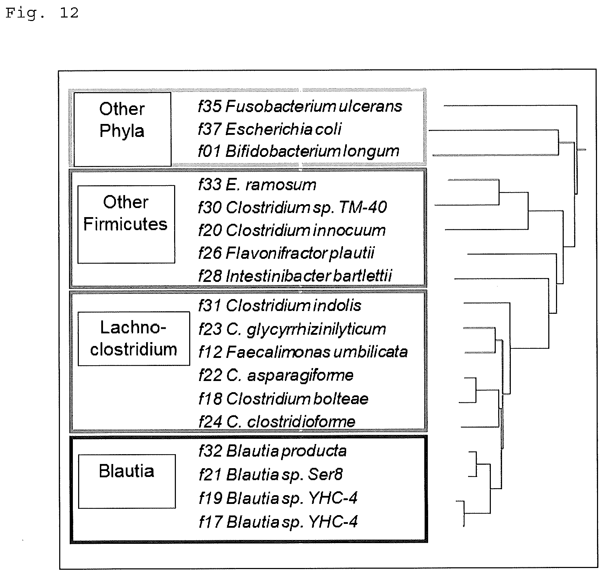

[Fig. 9] Fig. 9 is a phylogenetic tree showing a breakdown of F37mix, F8mix, and F29mix used in the experiment shown in Fig. 8. The phylogenetic tree was prepared by the Neighbor-joining method using MEGA X with the DNA base sequence of the 16S rDNA analysis result of the isolated bacteria by the Sanger method. The same applies to the preparation of the phylogenetic trees in Figs. 10 and 12.
[Fig. 10] Fig. 10 is a phylogenetic tree showing a breakdown of 18 bacterial strains derived from feces F (F18mix) .

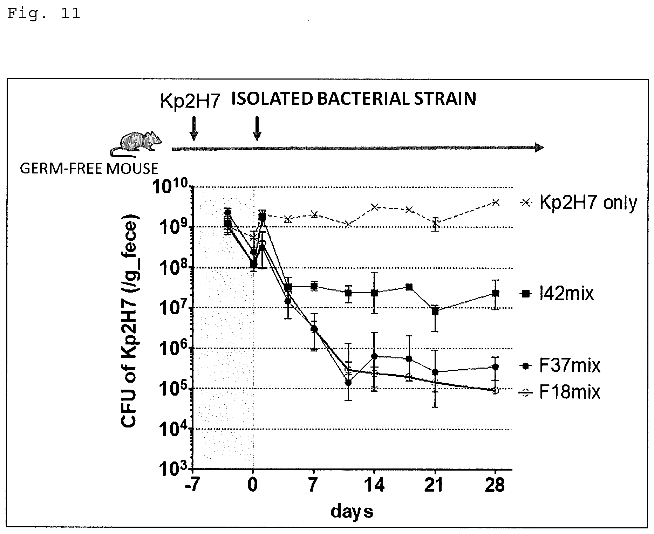

[Fig. 11] Fig. 11 is a graph in CFU showing changes over time in the bacterial amount of fecal Kp2H7 when Kp2H7 was colonized in germ-free mice, and then 37 bacterial strains derived from feces F (F37mix described above), 18 bacterial strains derived from feces F (F18mix shown in Fig. 10), or 42 bacterial strains derived from the feces derived from healthy subject I (feces I) were mixed and administered. F18mix also successfully eliminated Klebsiella in the same way as F37mix.
[Fig. 12] Fig. 12 is a phylogenetic tree that classifies 18 bacterial strains derived from feces F (F18mix) into 4 groups and shows the breakdown thereof. These 4 groups were subtracted from the 18 bacterial strains (F18mix) to prepare bacterial strain groups of F15mix (F18mix-other phyla), F12mix (Fl8mix-Lachnoclostridium), F14mix (F18mix-Blautia), and F13mix (F18mix-other Firmicutes), which were subjected to the experiment shown in Fig. 11.

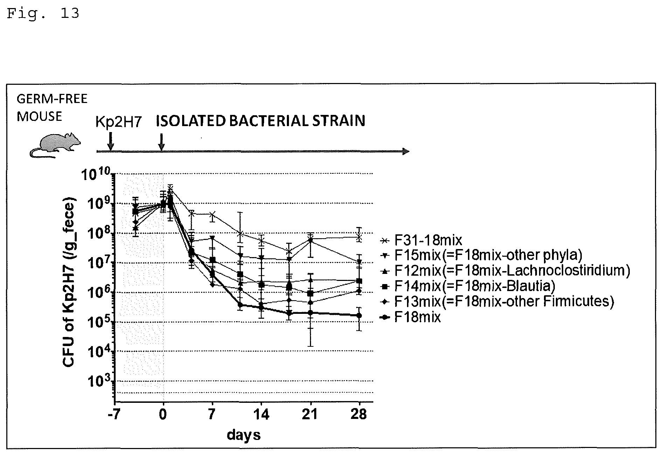

[Fig. 13] Fig. 13 is a graph in CFU showing changes over time in the bacterial amount of fecal Kp2H7 when Kp2H7 was colonized in germ-free mice, and then the group excluding the duplicate strains and F18mix from F37mix (F31-18mix), F15mix described above, F12mix described above, F14mix described above, F13mix described above, or F18mix described above was mixed and administered. Note that Fig. 13 shows data from the experiments performed twice. Excluding any group shown in Fig. 12 from F18mix decreased the ability to eliminate Klebsiella, and it has become clear that every group is important for the elimination of Klebsiella.

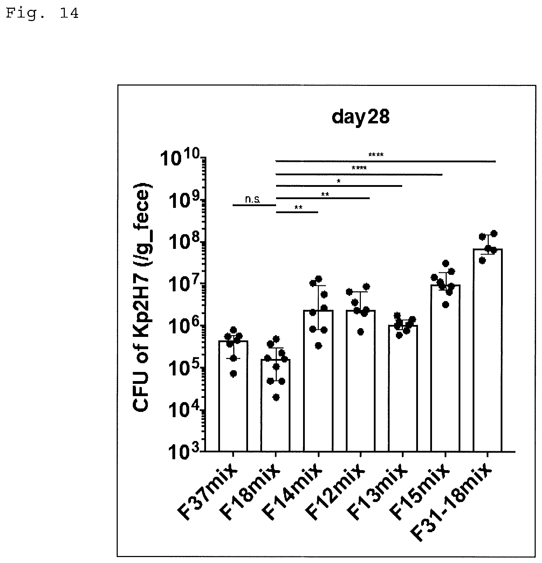

[Fig. 14] Fig. 14 is a graph showing the CFU of fecal Kp2H7 in each group at the time of day 28 in the experiment shown in Fig. 13. The F18mix-administered group has a statistically significantly smaller bacterial amount of Klebsiella than the other administration groups excluding F37mix.

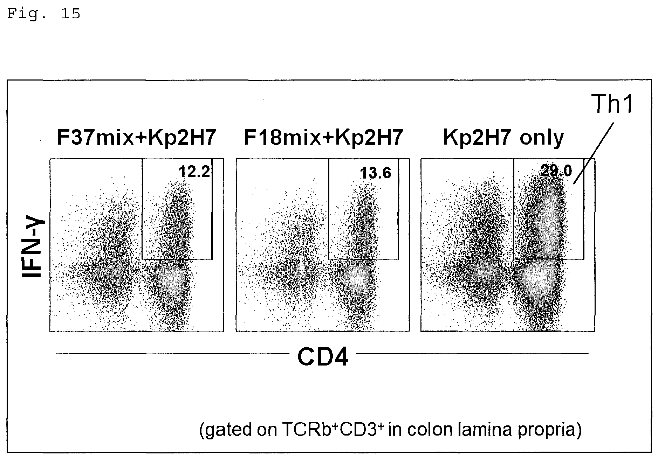

[Fig. 15] Fig. 15 is a dot plot diagram showing the results of flow cytometric analysis of immune cells in the lamina propria of the large intestine of mice in the F37mix-administered group, the F18mix-administered group, or the Kp2H7 alone administration group. The numerical value in each gate (square) in the figure shows the proportion of CD4+IFNy+ cells. The induction of CD4+IFNy+ cells is suppressed in the F37mix-administered group and the Fl8mix-administered group as compared with the Kp2H7 alone administration group.

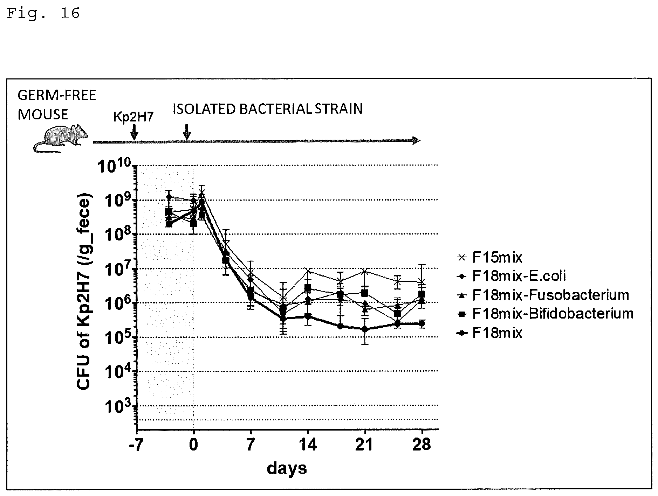

[Fig. 16] Fig. 16 is a graph in CFU showing changes over time in the bacterial amount of fecal Kp2H7 when Kp2H7 was administered to germ-free mice, and one week later, each bacterial strain mix was administered. The "F15mix" in the figure shows the result of mice administered by removing three strains of E. coli, Fusobacterium, and Bifidobacterium from F18mix, and "Fl8mix-E. coli", "F18mix-Fusobacterium," and "F18mix-Bifidobacterium" each show the result of mice administered by removing one of the three strains from F18mix. The effects of all three strains diminished when removed from F18mix, indicating that each was involved in the elimination of Klebsiella.

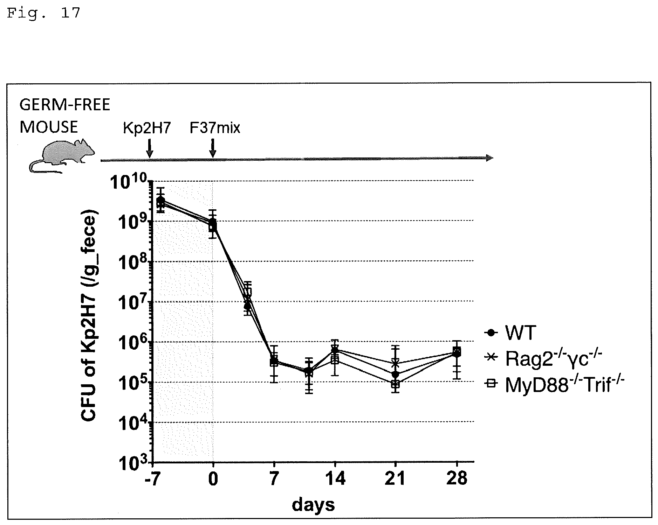

[Fig. 17] Fig. 17 is a graph in CFU showing changes over time in the bacterial amount of fecal Kp2H7 when Kp2H7 was administered to a germ-free Rag2⁻/⁻γc⁻/⁻ mouse, MyD88⁻/⁻ Triff⁻/⁻ mouse, or wild-type mouse (WT), and one week later, mixed F37mix was administered. Klebsiella was equally eliminated in all types of mice. This suggests that the major innate immunity and acquired immunity of the host are not involved in the elimination of Klebsiella.

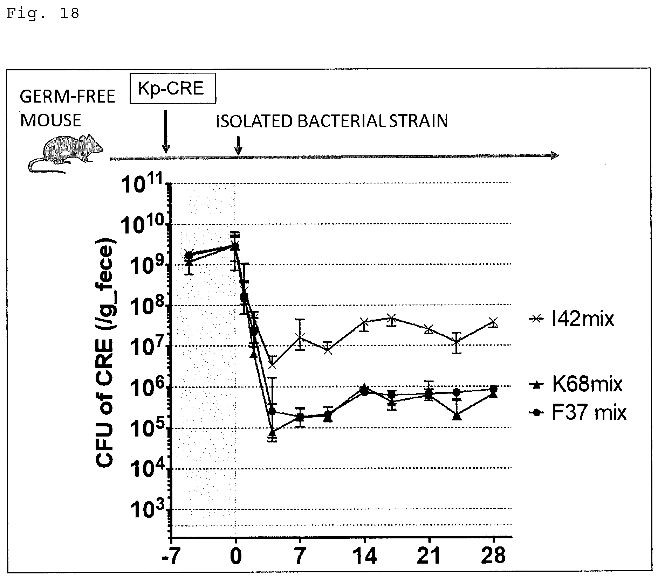

[Fig. 18] Fig. 18 is a graph in CFU showing changes over time in the bacterial amount of fecal CRE when Klebsiella (Kp-CRE) was administered to germ-free mice, and one week later, the isolated bacteria mixes (F37mix, K68mix, I42mix) were administered to the mice. F37mix and K68mix can also reduce CRE.

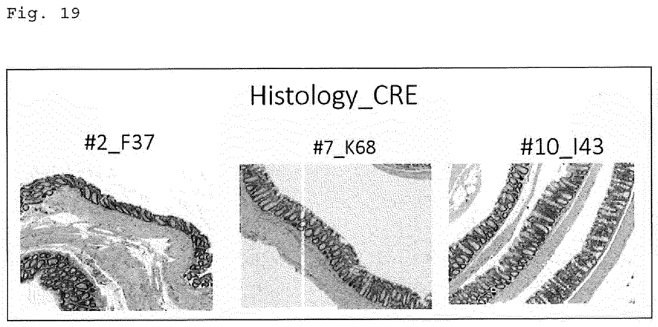

[Fig. 19] Fig. 19 is a photomicrograph showing the results of analyzing the large intestine of a mouse at the end of the experiment shown in Fig. 18 by HE staining. No inflammatory findings were observed in any of the isolated bacterium mix-administered mice.

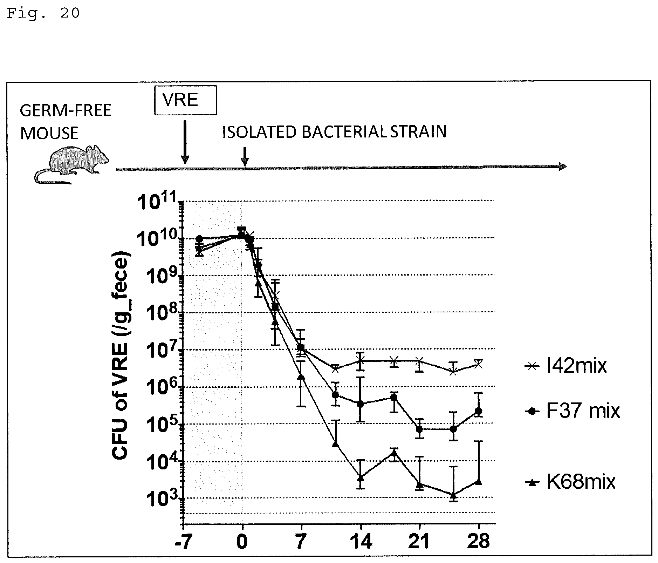

[Fig. 20] Fig. 20 is a graph in CFU showing changes over time in the bacterial amount of fecal VRE when VRE (vancomycin-resistant enterococci) was administered to germ-free mice, and one week later, the isolated bacteria mixes (F37mix, K68mix, I42mix) were administered to the mice. For VRE, K68mix was able to reduce the bacterial amount more than F37mix.

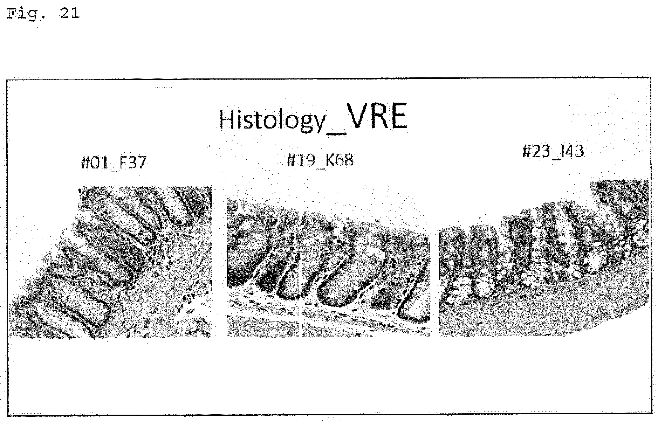

[Fig. 21] Fig. 21 is a photomicrograph showing the results of analyzing the large intestine of a mouse at the end of the experiment shown in Fig. 18 by HE staining. No inflammatory findings were observed in any of the isolated bacterium mix-administered mice.

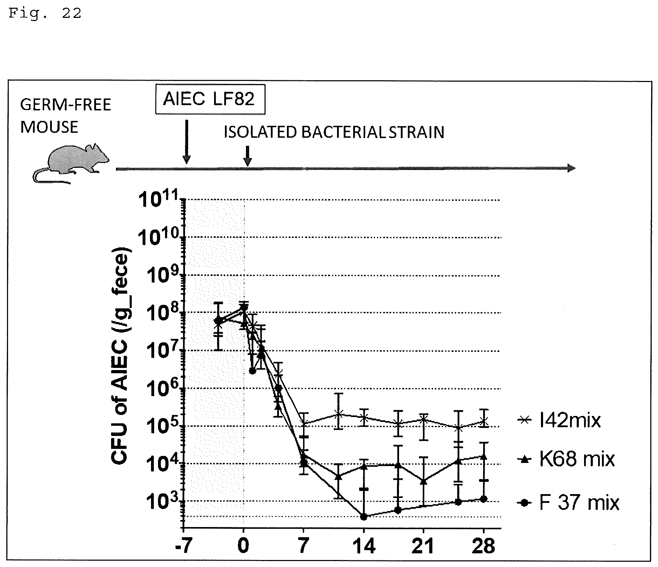

[Fig. 22] Fig. 22 is a graph in CFU showing changes over time in the bacterial amount of fecal AIEC when AIEC was administered to germ-free mice, and one week later, the isolated bacteria mixes (F37mix, K68mix, I42mix) were administered to the mice. For AIEC, F37mix was most effective in reducing the bacterial amount.

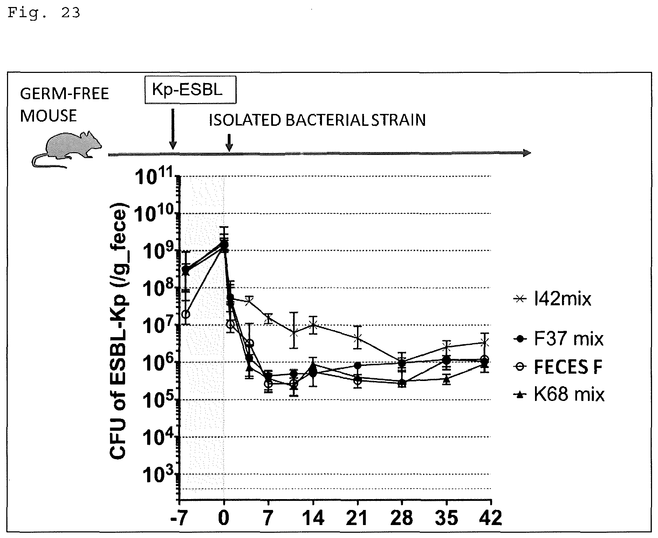

[Fig. 23] Fig. 23 is a graph in CFU showing changes over time in the bacterial amount of fecal ESBL-producing Klebsiella when ESBL-producing Klebsiella was administered to germ-free mice, and one week later, the isolated bacterium mixes (F37mix, K68mix, I42mix) were administered to the mice. F37mix and K68mix were able to eliminate ESBL-producing Klebsiella as much as F-derived feces.

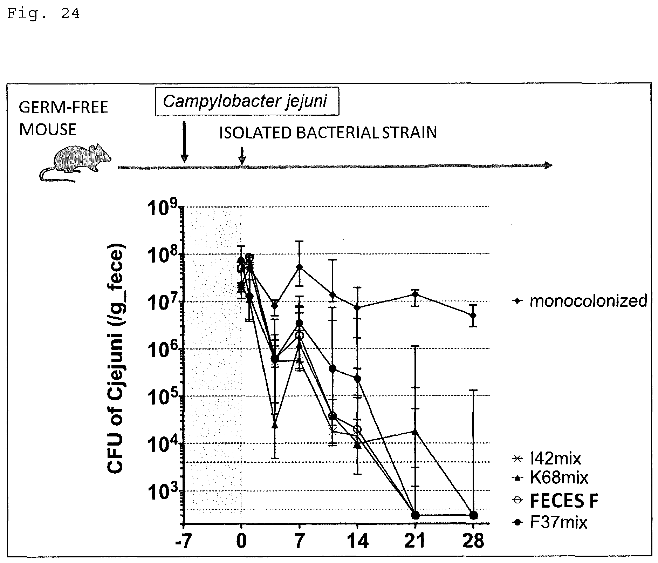

[Fig. 24] Fig. 24 is a graph in CFU showing changes over time in the bacterial amount of fecal Campylobacter when Campylobacter jejuni was administered to germ-free mice, and one week later, fecal samples derived from the isolated bacteria mixes (F37mix, K68mix, I42mix) or healthy subject F were administered to the mice. Elimination of Campylobacter jejuni was observed to the same extent in all the isolated bacteria mix-administered groups.

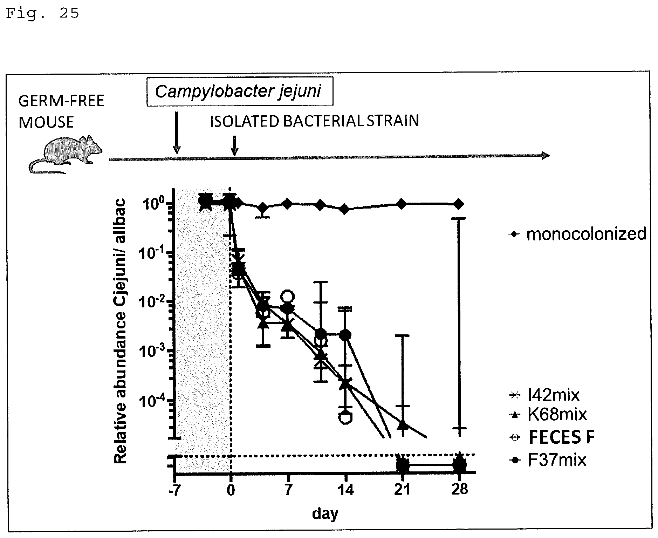

[Fig. 25] Fig. 25 is a graph showing changes over time in the bacterial amount of fecal Campylobacter as a relative value divided by the total bacterial amount when Campylobacter jejuni was administered to germ-free mice, and one week later, fecal samples derived from the isolated bacteria mixes (F37mix, K68mix, I42mix) or healthy subject F were administered to the mice. Elimination of Campylobacter jejuni was observed to the same extent in all the isolated bacteria mix-administered groups.

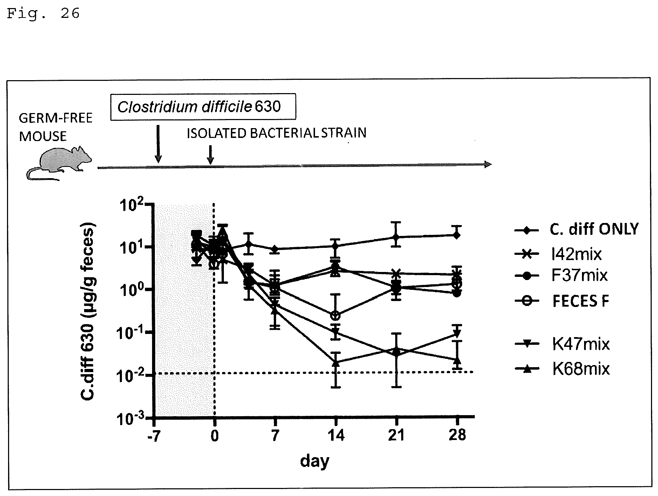

[Fig. 26] Fig. 26 is a graph showing the results of qPCR analysis of changes over time in the bacterial amount of fecal Clostridium difficile when Clostridium difficile was administered to germ-free mice, and one week later, fecal samples derived from the isolated bacteria mixes (F37mix, K68mix, I42mix, K47mix) or healthy subject F were administered to the mice. K68mix and K47mix were able to eliminate Clostridium difficile as compared with F-derived feces, but the elimination effect of F37mix was lower.

### [Description of Embodiments]

### <Intestinal Bacteria>

In the present invention, the intestinal bacteria contained as the only active ingredients of the antibacterial composition have an antibacterial action against drug-resistant bacteria or pro-inflammatory bacteria (hereinafter also referred to as "drug-resistant bacteria and the like") in the intestinal tract.

In the present invention, the "antibacterial activity" means an activity of suppressing the activity of bacteria, more specifically, an activity of suppressing the proliferation or colonization of bacteria or killing bacteria, and examples thereof include the activity of suppressing the colonization of bacteria in the intestines and the activity of eliminating bacteria from the intestines.

The "Intestinal bacteria" mean bacteria present in the intestinal tract of an animal. Examples of animals in which such bacteria are present include humans and non-human animals (such as mice, rats, monkeys, pigs, cattle, horses, sheep, goats, chickens, ducks, ostriches, domestic ducks, dogs, cats, rabbits, and hamsters), but among these animals, humans are preferable.

In the present invention, the "intestinal bacteria" may be a single strain of bacteria or a mixture of bacterial strains composed of multiple strains of bacteria. Note that when composed of multiple strains of bacteria, it is desirable that at least one of the bacterial strains has antibacterial activity against drug-resistant bacteria and the like. Further, in such a case, the multiple strains of bacteria may even include a bacterial strain that does not have the antibacterial activity, such as a bacterial strain having an action of enhancing the activity of a bacterial strain having the antibacterial activity, a bacterial strain having an action of maintaining the proliferation or colonization of a bacterial strain having antibacterial activity, and a bacterial strain having an action of suppressing the inhibitory activity of a bacterium that inhibits the antibacterial activity.

Examples of the "intestinal bacteria" include at least one bacterium having a DNA composed of a base sequence set forth in any of SEQ ID NOs: 1 to 147 or a base sequence having at least 70% identity to the base sequence, at least one bacterium having a DNA composed of a base sequence set forth in any of SEQ ID NOs: 1 to 68 or a base sequence having at least 70% identity to the base sequence, at least one bacterium having a DNA composed of a base sequence set forth in any of SEQ ID NOs: 69 to 105 or a base sequence having at least 70% identity to the base sequence (for example, at least one bacterium having a DNA composed of a base sequence set forth in any of SEQ ID NOs: 69, 80, 85 to 92, 94, 96, 98 to 101, 103, and 105 or a base sequence having at least 70% identity to the base sequence), and at least one bacterium having a DNA composed of a base sequence set forth in any of SEQ ID NOs: 106 to 147 or a base sequence having at least 70% identity to the base sequence.

The sequence indicated by each SEQ ID NO is the sequence of 16S rDNA of each bacterium K68, F37, and I43 in the attached document. Tables 1 to 4 below show the correspondence between each bacterium, the SEQ ID NO indicating the sequence of each 16S rDNA, and each bacterial species estimated from the sequence. In addition, K, F, and I represent intestinal bacteria isolated from the feces of three healthy subjects (Japanese) (see PTL 2).

**[Table 1]**

| No. | Species | SEQ ID NO: | subject id | NCBI TAX ID | % identity |
|---|---|---|---|---|---|
| K01 | Drancourtella massiliensis | 1 | NR_144722.1 | 1632013 | 99.57 |
| K02 | Bacteroides ovatus | 2 | NR_112940.1 | 28116 | 99.86 |
| K03 | Blautia coccoides | 3 | NR_104700.1 | 1532 | 99.86 |
| K04 | Blautia hominis | 4 | NR_163638.1 | 2025493 | 99.43 |
| K05 | Desulfovibrio vulgaris | 5 | NR_074446.1 | 882 | 91.58 |
| K06 | Alistipes onderdonkii | 6 | NR_043318.1 | 328813 | 99.86 |
| K07 | Eisenbergiella massiliensis | 7 | NR_144731.1 | 1720294 | 99.50 |
| K08 | [Clostridium] innocuum | 8 | NR_029164.1 | 1522 | 97.93 |
| K09 | Bacteroides fragilis | 9 | NR_112936.1 | 817 | 99.71 |
| K10 | Eggerthella lenta | 10 | NR_074377.1 | 84112 | 100.00 |
| K11 | Dielma fastidiosa | 11 | NR_125593.1 | 1034346 | 99.71 |
| K12 | Erysipelatoclostridium ramosum | 12 | NR_113243.1 | 1547 | 100.00 |
| K13 | Enterococcus faecalis | 13 | NR_115765.1 | 1351 | 99.93 |
| K14 | Bacteroides intestinalis | 14 | NR_041307.1 | 329854 | 99.35 |
| K15 | [Clostridium] symbiosum | 15 | NR_118730.1 | 1512 | 98.35 |
| K16 | [Clostridium] hylemonae | 16 | NR_024719.1 | 89153 | 99.50 |
| K17 | Hungatella effluvii | 17 | NR_133762.1 | 1096246 | 98.49 |
| K18 | Bacteroides dorei | 18 | NR_041351.1 | 357276 | 99.93 |
| K19 | [Clostridium] clostridioforme | 19 | NR_044715.2 | 1531 | 98.99 |
| K20 | Flavonifractor plautii | 20 | NR_029356.1 | 292800 | 100.00 |
| K21 | Bacteroides xylanisolvens | 21 | NR_112947.1 | 657309 | 99.50 |
| K22 | Bacteroides thetaiotaomicron | 22 | NR_112944.1 | 818 | 99.93 |
| K23 | Parabacteroides merdae | 23 | NR_041343.1 | 46503 | 99.78 |
| K24 | Bacteroides vulgatus | 24 | NR_074515.1 | 435590 | 100.00 |
| K25 | [Clostridium] aldenense | 25 | NR_043680.1 | 358742 | 99.41 |
| K26 | Bacteroides uniformis | 26 | NR_112945.1 | 820 | 97.39 |
| K27 | Gordonibacter urolithinfaciens | 27 | NR_148261.1 | 1335613 | 99.56 |
| K28 | Coprococcus comes | 28 | NR_044048.1 | 470146 | 92.77 |
| K29 | Anaerostipes caccae | 29 | NR_028915.1 | 105841 | 98.78 |
| K30 | [Ruminococcus] gnavus | 30 | NR_036800.1 | 411470 | 99.78 |
| K31 | [Ruminococcus] gnavus | 31 | NR_036800.1 | 411470 | 98.71 |
| K32 | Alistipes shahii | 32 | NR_113153.1 | 328814 | 100.00 |
| K33 | Bacteroides stercoris | 33 | NR_112943.1 | 449673 | 98.77 |
| K34 | Blautia hominis | 34 | NR_163634.1 | 2025493 | 98.71 |

**[Table 2]**

| No. | Species | SEQ ID NO: | subject id | NCBI TAX ID | % identity |
|---|---|---|---|---|---|
| K35 | Butyricicoccus faecihominis | 35 | NR_152060.1 | 1712515 | 97.89 |
| K36 | [Clostridium] bolteae | 36 | NR_025567.1 | 208479 | 99.50 |
| K37 | Phocea massiliensis | 37 | NR_144748.1 | 1841867 | 99.93 |
| K38 | Holdemania massiliensis | 38 | NR_125628.1 | 1211819 | 99.71 |
| K39 | Escherichia coli | 39 | NR_114042.1 | 562 | 99.79 |
| K40 | Agathobaculum desmolans | 40 | NR_044644.2 | 39484 | 96.68 |
| K41 | [Eubacterium] rectale | 41 | NR_074634.1 | 515619 | 100.00 |
| K42 | Lactonifactor longoviformis | 42 | NR_043551.1 | 341220 | 100.00 |
| K43 | Oscillibacter ruminantium | 43 | NR_118156.1 | 1007096 | 95.90 |
| K44 | Pseudoflavonifractor phocaeensis | 44 | NR_147370.1 | 1870988 | 97.42 |
| K45 | Streptococcus pasteurianus | 45 | NR_043660.1 | 197614 | 100.00 |
| K46 | Sutterella wadsworthensis | 46 | NR_117778.1 | 40545 | 99.93 |
| K47 | Bifidobacterium faecale | 47 | NR_133982.1 | 1454229 | 99.35 |
| K48 | Eisenbergiella massiliensis | 48 | NR_144731.1 | 1720294 | 93.50 |
| K49 | Fusicatenibacter saccharivorans | 49 | NR_114326.1 | 1150298 | 99.78 |
| K50 | Robinsoniella peoriensis | 50 | NR_041882.1 | 180332 | 93.40 |
| K51 | Massilimicrobiota timonensis | 51 | NR_144738.1 | 1776392 | 99.93 |
| K52 | Blautia faecis | 52 | NR_109014.1 | 871665 | 99.92 |
| K53 | Blautia wexlerae | 53 | NR_044054.1 | 1121115 | 98.63 |
| K54 | Phascolarctobacterium faecium | 54 | NR_026111.1 | 33025 | 99.72 |
| K55 | Odoribacter splanchnicus | 55 | NR_113075.1 | 28118 | 99.71 |
| K56 | Faecalibacterium prausnitzii | 56 | NR_028961.1 | 853 | 97.29 |
| K57 | Colidextribacter massiliensis | 57 | NR_147375.1 | 1870986 | 94.70 |
| K58 | Emergencia timonensis | 58 | NR_144737.1 | 1776384 | 91.61 |
| K59 | Alistipes finegoldii | 59 | NR_043064.1 | 214856 | 99.42 |
| K60 | Ruthenibacterium lactatiformans | 60 | NR_151900.1 | 1550024 | 99.93 |
| K61 | Christensenella minuta | 61 | NR_112900.1 | 626937 | 98.71 |
| K62 | [Clostridium] scindens | 62 | NR_028785.1 | 29347 | 98.49 |
| K63 | Enterococcus faecalis | 63 | NR_115765.1 | 1351 | 100.00 |
| K64 | Blautia coccoides | 64 | NR_104700.1 | 1532 | 99.93 |
| K65 | Alistipes ihumii | 65 | NR_144706.1 | 1211813 | 99.28 |
| K66 | Intestinimonas butyriciproducens | 66 | NR_118554.1 | 1297617 | 99.86 |
| K67 | Bacteroides uniformis | 67 | NR_112945.1 | 820 | 99.93 |
| K68 | Akkermansia muciniphila | 68 | NR_074436.1 | 239935 | 98.91 |

**[Table 3]**

| No. | Species | SEQ ID NO: | subject id | NCBI TAX ID | % identity |
|---|---|---|---|---|---|
| F01 | Bifidobacterium longum | 69 | NR_145535.1 | 1931217 | 99.60 |
| F02 | Bacteroides xylanisolvens | 70 | NR_112947.1 | 657309 | 99.59 |
| F03 | Bacteroides fragilis | 71 | NR_074784.2 | 817 | 98.95 |
| F04 | Bacteroides uniformis | 72 | NR_112945.1 | 820 | 99.93 |
| F05 | Bacteroides thetaiotaomicron | 73 | NR_074277.1 | 818 | 99.80 |
| F06 | Bacteroides uniformis | 74 | NR_112945.1 | 820 | 99.93 |
| F07 | Bacteroides acidifaciens | 75 | NR_112931.1 | 85831 | 97.18 |
| F08 | Bacteroides fragilis | 76 | NR_074784.2 | 817 | 98.95 |
| F09 | Parabacteroides goldsteinii | 77 | NR_113076.1 | 927665 | 98.98 |
| F10 | [Ruminococcus] gnavus | 78 | NR_036800.1 | 411470 | 99.80 |
| F11 | Blautia luti | 79 | NR_114315.1 | 649762 | 97.57 |
| F12 | Faecalimonas umbilicata | 80 | NR_156907.1 | 1912855 | 99.87 |
| F13 | [Clostridium] saccharolyticum | 81 | NR_102852.1 | 84030 | 94.34 |
| F14 | Tyzzerella nexilis | 82 | NR_029248.1 | 500632 | 98.49 |
| F15 | [Ruminococcus] gnavus | 83 | NR_036800.1 | 411470 | 99.80 |
| F16 | Anaerostipes hadrus | 84 | NR_117138.2 | 649756 | 99.74 |
| F17 | Blautia glucerasea | 85 | NR_113231.1 | 536633 | 96.91 |
| F18 | [Clostridium] bolteae | 86 | NR_025567.1 | 208479 | 99.60 |
| F19 | Blautia caecimuris | 87 | NR_144607.1 | 1796615 | 99.13 |
| F20 | [Clostridium] innocuum | 88 | NR_029164.1 | 1522 | 98.71 |
| F21 | Blautia marasmi | 89 | NR_147395.1 | 1917868 | 98.20 |
| F22 | [Clostridium] lavalense | 90 | NR_044289.1 | 460384 | 98.97 |
| F23 | [Clostridium] glycyrrhizinilyticum | 91 | NR_112553.1 | 342942 | 99.13 |
| F24 | Eisenbergiella massiliensis | 92 | NR_144731.1 | 1720294 | 97.73 |
| F25 | [Clostridium] glycyrrhizinilyticum | 93 | NR_112553.1 | 342942 | 99.13 |
| F26 | Flavonifractor plautii | 94 | NR_029356.1 | 292800 | 100.00 |
| F27 | Blautia luti | 95 | NR_114315.1 | 649762 | 97.57 |
| F28 | Intestinibacter bartlettii | 96 | NR_027573.1 | 261299 | 99.93 |
| F29 | [Ruminococcus] gnavus | 97 | NR_036800.1 | 411470 | 98.85 |
| F30 | Massilimicrobiota timonensis | 98 | NR_144738.1 | 1776392 | 100.00 |
| F31 | Anaerostipes caccae | 99 | NR_028915.1 | 105841 | 97.32 |
| F32 | Blautia coccoides | 100 | NR_104700.1 | 1532 | 99.93 |
| F33 | Erysipelatoclostridium ramosum | 101 | NR_029247.1 | 1547 | 99.41 |
| F34 | Veillonella parvula | 102 | NR_074980.1 | 479436 | 99.16 |
| F35 | Fusobacterium varium | 103 | NR_113384.1 | 856 | 99.73 |
| F36 | Fusobacterium varium | 104 | NR_113384.1 | 856 | 99.73 |
| F37 | Escherichia fergusonii | 105 | NR_074902.1 | 585054 | 99.87 |

**[Table 4]**

| No. | Species | SEQ ID NO: | subject id | NCBI TAX ID | % identity |
|---|---|---|---|---|---|
| I01 | Bifidobacterium faecale | 106 | NR_133982.1 | 1454229 | 98.06 |
| I02 | Bifidobacterium pseudocatenulatum | 107 | NR_037117.1 | 28026 | 99.63 |
| I03 | Bifidobacterium bifidum | 108 | NR_044771.1 | 1681 | 100.00 |
| I04 | Bifidobacterium longum | 109 | NR_145535.1 | 1931217 | 99.70 |
| I05 | Collinsella aerofaciens | 110 | NR_113316.1 | 74426 | 99.92 |
| I06 | Collinsella aerofaciens | 111 | NR_113316.1 | 74426 | 99.72 |
| I07 | Bifidobacterium longum | 112 | NR_145535.1 | 1931217 | 99.70 |
| I08 | Bacteroides stercoris | 113 | NR_112943.1 | 449673 | 99.18 |
| I09 | Bacteroides massiliensis | 114 | NR_042745.1 | 204516 | 99.70 |
| I10 | Bacteroides vulgatus | 115 | NR_074515.1 | 435590 | 100.00 |
| I11 | Bacteroides dorei | 116 | NR_041351.1 | 357276 | 100.00 |
| I12 | Parabacteroides merdae | 117 | NR_041343.1 | 46503 | 99.85 |
| I13 | Parabacteroides distasonis | 118 | NR_041342.1 | 823 | 99.03 |
| I14 | Alistipes putredinis | 119 | NR_113152.1 | 28117 | 100.00 |
| I15 | Bacteroides uniformis | 120 | NR_040866.1 | 820 | 97.75 |
| I16 | Bacteroides koreensis | 121 | NR_159117.1 | 1912896 | 99.26 |
| I17 | Alistipes shahii | 122 | NR_113153.1 | 328814 | 99.63 |
| I18 | Odoribacter splanchnicus | 123 | NR_113075.1 | 28118 | 99.77 |
| I19 | Faecalibacterium prausnitzii | 124 | NR_028961.1 | 853 | 97.24 |
| I20 | Faecalibacterium prausnitzii | 125 | NR_028961.1 | 853 | 97.24 |
| I21 | Blautia wexlerae | 126 | NR_044054.1 | 1121115 | 98.38 |
| I22 | Ruminococcus lactaris | 127 | NR_027579.1 | 471875 | 97.24 |
| I23 | Ruminococcus albus | 128 | NR_113032.1 | 1264 | 94.68 |
| I24 | Faecalibacterium prausnitzii | 129 | NR_028961.1 | 853 | 96.86 |
| I25 | Dorea longicatena | 130 | NR_028883.1 | 88431 | 99.47 |
| I26 | Dorea formicigenerans | 131 | NR_044645.2 | 39486 | 97.94 |
| I27 | Anaerostipes hadrus | 132 | NR_117138.2 | 649756 | 99.85 |
| I28 | Intestinibacter bartlettii | 133 | NR_027573.1 | 261299 | 99.55 |
| I29 | Flavonifractor plautii | 134 | NR_029356.1 | 292800 | 99.71 |
| I30 | Pseudoflavonifractor phocaeensis | 135 | NR_147370.1 | 1870988 | 97.86 |
| I31 | [Clostridium] spiroforme | 136 | NR_114393.1 | 29348 | 93.09 |
| I32 | Megasphaera elsdenii | 137 | NR_102980.1 | 1064535 | 99.35 |
| I33 | Dialister succinatiphilus | 138 | NR_041666.1 | 742743 | 97.02 |
| I34 | Acidaminococcus intestini | 139 | NR_041894.1 | 187327 | 99.93 |
| I35 | Allisonella histaminiformans | 140 | NR_028862.1 | 209880 | 99.50 |
| I36 | Megasphaera massiliensis | 141 | NR_133027.1 | 1232428 | 99.00 |
| I37 | Sutterella wadsworthensis | 142 | NR_104851.1 | 40545 | 99.78 |
| I38 | Clostridium baratii | 143 | NR_029229.1 | 1561 | 99.85 |
| I39 | Anaeromassilibacillus senegalensis | 144 | NR_144727.1 | 1673717 | 97.27 |
| I40 | Colidextribacter massiliensis | 145 | NR_147375.1 | 1870986 | 95.00 |
| I41 | Flavonifractor plautii | 146 | NR_029356.1 | 292800 | 97.96 |
| I42 | [Clostridium] leptum | 147 | NR_114789.1 | 1535 | 94.55 |

Tables 1 to 4 show the top-hit species names and RefSeq accessions as a result of a BLAST search for the sequences set forth in the SEQ ID NOs on the RefSeq 16s DNA sequence database (as of January 8, 2020). Note that it is generally said %identity>97% can identify species, and >94% can identify genus. Therefore, it should be understood that bacterial strains with a %identity of >94% are bacteria that can be identified at the genus level.

The "at least 70% identity" in the intestinal bacteria of the present invention means that the identity to each base sequence is preferably 80% or more, more preferably 85% or more, further preferably 90% or more (for example, 91% or more, 92% or more, 93% or more, and 94% or more), more preferably 94% or more (for example, 95% or more, 96% or more, 97% or more, and 98% or more), and particularly preferably 99% or more.

In addition, the homology or identity of a sequence (amino acid sequence or nucleotide (base) sequence) can be determined by using the BLAST (Basic Local Alignment Search) program (Altschul et al. J. Mol. Biol., 215: 403-410, 1990). The program is based on the algorithm BLAST by Karlin and Altschul (Proc. Natl. Acad. Sci. USA, 87: 2264-2268, 1990, Proc. Natl. Acad. Sci. USA, 90: 5873-5877, 1993). When analyzing the homology or identity between sequences by BLAST, it can be determined by using, for example, the BLAST of the National Center for Biotechnology Information (NCBI) (for example, by using the default, that is, initially set parameters).

The "intestinal bacterium" which includes a DNA composed of a base sequence set forth in any of SEQ ID NOs: 1 to 147 or a base sequence having at least 70% identity to the base sequence is preferably at least 15 bacteria in the intestinal bacterial group, more preferably at least 30 bacteria in the intestinal bacterial group, further preferably at least 75 bacteria in the intestinal bacterial group, more preferably at least 120 bacteria in the intestinal bacterial group, further preferably at least 135 bacteria in the intestinal bacterial group, more preferably at least 140 bacteria in the intestinal bacterial group, further preferably 147 intestinal bacteria each of which includes a DNA composed of a base sequence set forth in any of SEQ ID NOs: 1 to 147 or a base sequence having at least 70% identity to the base sequence, and particularly preferably 147 bacteria each of which includes a DNA composed of a base sequence set forth in any of SEQ ID NOs: 1 to 147.

The "intestinal bacterium" which includes a DNA composed of a base sequence set forth in any of SEQ ID NOs: 1 to 68 or a base sequence having at least 70% identity to the base sequence is preferably at least 7 bacteria in the intestinal bacterial group, more preferably at least 15 bacteria in the intestinal bacterial group, further preferably at least 35 bacteria in the intestinal bacterial group, more preferably at least 60 bacteria in the intestinal bacterial group, further preferably at least 65 bacteria in the intestinal bacterial group, more preferably 68 intestinal bacteria each of which includes a DNA composed of a base sequence set forth in any of SEQ ID NOs: 1 to 68 or a base sequence having at least 70% identity to the base sequence, and particularly preferably 68 bacteria each of which includes a DNA composed of a base sequence set forth in any of SEQ ID NOs: 1 to 68. In addition, the "intestinal bacterium" which includes a DNA composed of a base sequence set forth in any of SEQ ID NOs: 1 to 68 or a base sequence having at least 70% identity to the base sequence desirably has resistance to ampicillin. Also, 46 bacteria each of which includes a DNA composed of a base sequence set forth in any of SEQ ID NOs: 1 to 46 or a base sequence having at least 70% identity to the base sequence can be used.

The "intestinal bacterium" which includes a DNA composed of a base sequence set forth in any of SEQ ID NOs: 69 to 105 or a base sequence having at least 70% identity to the base sequence is preferably at least 4 bacteria in the intestinal bacterial group, more preferably at least 8 bacteria in the intestinal bacterial group, further preferably at least 18 bacteria in the intestinal bacterial group, more preferably at least 29 bacteria in the intestinal bacterial group, further preferably at least 33 bacteria in the intestinal bacterial group, more preferably at least 35 bacteria in the intestinal bacterial group, further preferably 37 intestinal bacteria each of which includes a DNA composed of a base sequence set forth in any of SEQ ID NOs: 69 to 105 or a base sequence having at least 70% identity to the base sequence, and particularly preferably 37 bacteria each of which includes a DNA composed of a base sequence set forth in any of SEQ ID NOs: 69 to 105. In addition, the "intestinal bacterium" which includes a DNA composed of a base sequence set forth in any of SEQ ID NOs: 69 to 105 or a base sequence having at least 70% identity to the base sequence desirably has sensitivity to ampicillin.

The "intestinal bacterium" which includes a DNA composed of a base sequence set forth in any of SEQ ID NOs: 69, 80, 85 to 92, 94, 96, 98 to 101, 103, and 105 or a base sequence having at least 70% identity to the base sequence is preferably at least 2 bacteria in the intestinal bacterial group, more preferably at least 5 bacteria in the intestinal bacterial group, further preferably at least 10 bacteria in the intestinal bacterial group, more preferably at least 14 bacteria in the intestinal bacterial group, further preferably at least 15 bacteria in the intestinal bacterial group, more preferably at least 16 bacteria in the intestinal bacterial group, further preferably at least 17 bacteria in the intestinal bacterial group, more preferably 18 intestinal bacteria each of which includes a DNA composed of a base sequence set forth in any of SEQ ID NOs: 69, 80, 85 to 92, 94, 96, 98 to 101, 103, and 105 or a base sequence having at least 70% identity to the base sequence, and particularly preferably 18 bacteria each of which includes a DNA composed of a base sequence set forth in any of SEQ ID NOs: 69, 80, 85 to 92, 94, 96, 98 to 101, 103, and 105 (bacteria which include a DNA composed of a base sequence set forth in SEQ ID NO: 69, bacteria which include a DNA composed of a base sequence set forth in SEQ ID NO: 80, bacteria which include a DNA composed of a base sequence set forth in SEQ ID NO: 85, bacteria which include a DNA composed of a base sequence set forth in SEQ ID NO: 86, bacteria which include a DNA composed of a base sequence set forth in SEQ ID NO: 87, bacteria which include a DNA composed of a base sequence set forth in SEQ ID NO: 88, bacteria which include a DNA composed of a base sequence set forth in SEQ ID NO: 89, bacteria which include a DNA composed of a base sequence set forth in SEQ ID NO: 90, bacteria which include a DNA composed of a base sequence set forth in SEQ ID NO: 91, bacteria which include a DNA composed of a base sequence set forth in SEQ ID NO: 92, bacteria which include a DNA composed of a base sequence set forth in SEQ ID NO: 94, bacteria which include a DNA composed of a base sequence set forth in SEQ ID NO: 96, bacteria which include a DNA composed of a base sequence set forth in SEQ ID NO: 98, bacteria which include a DNA composed of a base sequence set forth in SEQ ID NO: 99, bacteria which include a DNA composed of a base sequence set forth in SEQ ID NO: 100, bacteria which include a DNA composed of a base sequence set forth in SEQ ID NO: 101, bacteria which include a DNA composed of a base sequence set forth in SEQ ID NO: 103, and bacteria which include a DNA composed of a base sequence set forth in SEQ ID NO: 105).

Note that typical examples of 18 bacteria each of which includes a DNA composed of a base sequence set forth in any of SEQ ID NOs: 69, 80, 85 to 92, 94, 96, 98 to 101, 103, and 105 are accession bacterial strains shown in Table 5 below. All of the bacterial strains were deposited with the National Institute of Technology and Evaluation (NITE, 2-5-8 Kazusakamatari, Kisarazu City, Chiba Prefecture, Room 122) on March 2, 2020.

**[Table 5]**

| No. | 16S rDNA (SEQ ID NO:) | Identification Indication | Receipt Number | Accession Number |
|---|---|---|---|---|
| F01 | 69 | f01_42H6 | NITE ABP-03147 | NITE BP-03147 |
| F12 | 80 | f12_42H4 | NITE ABP-03148 | NITE BP-03148 |
| F17 | 85 | f17_4217 | NITE ABP-03149 | NITE BP-03149 |
| F18 | 86 | f18_42I2 | NITE ABP-03150 | NITE BP-03150 |
| F19 | 87 | f19_43G2 | NITE ABP-03151 | NITE BP-03151 |
| F20 | 88 | f20_43G1 | NITE ABP-03152 | NITE BP-03152 |
| F21 | 89 | f21_42A8 | NITE ABP-03153 | NITE BP-03153 |
| F22 | 90 | f22_43C3 | NITE ABP-03154 | NITE BP-03154 |
| F23 | 91 | f23_42K4 | NITE ABP-03155 | NITE BP-03155 |
| F24 | 92 | f24_42I4 | NITE ABP-03156 | NITE BP-03156 |
| F26 | 94 | f26_42K2 | NITE ABP-03157 | NITE BP-03157 |
| F28 | 96 | f28_43A3 | NITE ABP-03158 | NITE BP-03158 |
| F30 | 98 | f30_43A5 | NITE ABP-03159 | NITE BP-03159 |
| F31 | 99 | f31_43J5 | NITE ABP-03160 | NITE BP-03160 |
| F32 | 100 | f32_42A7 | NITE ABP-03161 | NITE BP-03161 |
| F33 | 101 | f33_43N2 | NITE ABP-03162 | NITE BP-03162 |
| F35 | 103 | f35_42L8 | NITE ABP-03163 | NITE BP-03163 |
| F37 | 105 | f37_42G1 | NITE ABP-03164 | NITE BP-03164 |

Note that as long as the antibacterial action and the like against drug-resistant bacteria or pro-inflammatory bacteria are not impaired, the intestinal bacteria according to the present invention also include bacteria (such as derivative strains and inducible strains) bred from each of these bacteria by mutation treatment, genetic recombination, genome editing, selection of natural mutant strains, and the like.

The "intestinal bacterium" which includes a DNA composed of a base sequence set forth in any of SEQ ID NOs: 106 to 147 or a base sequence having at least 70% identity to the base sequence is preferably at least 4 bacteria in the intestinal bacterial group, more preferably at least 9 bacteria in the intestinal bacterial group, further preferably at least 22 bacteria in the intestinal bacterial group, more preferably at least 34 bacteria in the intestinal bacterial group, further preferably at least 39 bacteria in the intestinal bacterial group, more preferably at least 41 bacteria in the intestinal bacterial group, further preferably 42 intestinal bacteria each of which includes a DNA composed of a base sequence set forth in any of SEQ ID NOs: 106 to 147 or a base sequence having at least 70% identity to the base sequence, and particularly preferably 42 bacteria each of which includes a DNA composed of a base sequence set forth in any of SEQ ID NOs: 106 to 147. In addition, the "intestinal bacterium" which includes a DNA composed of a base sequence set forth in any of SEQ ID NOs: 106 to 147 or a base sequence having at least 70% identity to the base sequence desirably has sensitivity to ampicillin.

Further, the "intestinal bacterium" is an intestinal bacterium that exhibits resistance to at least one compound selected from the group consisting of spectinomycin, and/or sensitivity to at least one compound selected from the group consisting of ampicillin, tylosin, and chloroform. In addition, another aspect includes intestinal bacteria that exhibit resistance to metronidazole and/or sensitivity to at least one compound selected from the group consisting of vancomycin and tylosin.

In addition, as shown in Examples described later, the above-mentioned intestinal bacteria have been isolated by the present inventors and are useful because they exert an antibacterial action against drug-resistant bacteria, pro-inflammatory bacteria, and the like. Therefore, the present invention is defined in the claims.

### <Antibacterial Composition and pharmaceutical Composition>

The composition of the present invention may be any as long as it contains the 18 intestinal bacteria as the only active ingredients as defined in claims, and the bacterium may be a live bacterium or a dead bacterium. In addition, when the compositions can be used in combination, and they are ingested or absorbed in combination as a result (in the case of combined compositions), the above-mentioned intestinal bacteria can also be present separately in two or more compositions.

The composition of the present invention can be in the form of a pharmaceutical composition, a quasi-drug composition, food and drink (including animal feed), or a reagent used for research purposes (such as in vitro or in vivo experiments).

Since the composition of the present invention exhibits antibacterial activity against drug-resistant bacteria and the like, it is preferably used as a pharmaceutical composition, a quasi-drug composition, or food or drink for treating, preventing, or ameliorating diseases caused by the bacterium.

The composition of the present invention can be formulated by a known pharmaceutical method. For example, it can be used in the form of capsules, tablets, pills, liquids, powders, granules, fine granules, film coating preparations, pellets, troches, sublingual preparations, chews, buccal preparations, pastes, syrups, suspensions, elixirs, emulsions, coating preparations, ointments, platers, poultices, transdermal preparations, lotions, inhalants, aerosols, injections, suppositories, and the like, for the purpose of administration by oral, parenteral (for example, intestinal, intramuscular, intravenous, tracheal, intranasal, transdermal, intradermal, subcutaneous, intraocular, vaginal, intraperitoneal, rectal, or inhalation), or multiple combinations of these routes.

In these formulations, they can be appropriately combined with a pharmacologically or food and drink acceptable carrier, specifically, sterilized water, saline, buffer solution, medium, vegetable oil, solvent, base, emulsifier, suspension, surfactant, stabilizer, flavor agent, air freshener, excipient, vehicle, antiseptic, binder, diluent, isotonic agent, soothing agent, bulking agent, disintegrant, buffer agent, coating agent, lubricant, colorant, sweetener, thickener, flavor modifier, solubilizer, or other additives.

In addition, in these formulations, from the viewpoint of e.g., more efficiently exerting antibacterial activity against drug-resistant bacteria and the like in the intestinal tract, particularly in a formulation intended for oral administration, the composition of the present invention may be combined with a composition that enables efficient delivery into the intestinal tract. The composition capable of such delivery into the intestinal tract is not particularly limited, and a known composition can be appropriately employed, and examples thereof include pH-sensitive compositions, compositions that suppress release to the intestinal tract (such as cellulosic polymers, acrylic acid polymers and copolymers, and vinyl acid polymers and copolymers), bioadhesive compositions that specifically adhere to the intestinal mucosa (such as polymers described in US Patent No. 6.368.586), protease inhibitor-containing compositions, and compositions specifically degraded by intestinal enzymes).

In addition, when the antibacterial composition of the present invention is used as a pharmaceutical composition, it may further contain a known substance used for treating, preventing, or ameliorating diseases caused by drug-resistant bacteria and the like (such as other antibacterial agents, anti-inflammatory agents, and immunosuppressants), or may be used in combination with such a substance.

When the composition of the present invention is used as food and drink, the food and drink may be, for example, a health food, a functional food, a food for specified health use, a food with nutrient function claims, a food with functional claims, a dietary supplement, a food for patients, or animal feed. Specific examples of the food and drink include liquid foods such as fermented beverages, oil-containing products, soups, milk beverages, refreshing beverages, tea beverages, alcoholic beverages, energy drinks, and jelly-like beverages, carbohydrate-containing foods, processed livestock foods, and processed fishery foods; and processed vegetable foods, semi-solid foods, fermented foods, confectionery, retort products, microwave-compatible foods, and the like. It is also possible to list healthy food and drink prepared in the form of powder, granules, tablets, capsules, liquid, paste, or jelly. The food and drink according to the present invention can be produced by a production technique known in the art. In the food and drink, components (such as nutrients) effective for ameliorating or preventing diseases caused by drug-resistant bacteria and the like may be added. In addition, it may be a multifunctional food and drink by combining it with other ingredients or other functional foods that exhibit functions other than the amelioration and the like.

The product (pharmaceutical product, quasi-drug, food and drink, reagent) of the composition of the present invention or the instruction manual thereof may be provided with an indication that it exhibits antibacterial activity against drug-resistant bacteria and the like, or is used for treating, ameliorating, or preventing diseases caused by the drug-resistant bacteria and the like. Further, in relation to the food and drink, the product or the like of the composition of the present invention may be labeled with a health function as a food with health claims (food for specified health use, food with nutrient function claims, food with functional claims) so that the form, target, and the like can be distinguished from general foods. Here, "the product or the instruction manual is labeled" means that a label is attached to the main body, container, packaging, or the like of the product, or a label is attached to the instruction manual, package insert, promotional material, other printed matter, or the like that discloses product information. Further, the composition of the present invention may be in the form of a kit.

Further, as described above, a pharmaceutical composition can be produced by a known formulation technique using the intestinal bacterium and the like of the present invention. Therefore, the present invention also provides the pharmaceutical composition for use in treating, ameliorating, or preventing diseases caused by drug-resistant bacteria

### <Medical use>

The present invention also provides the antibacterial composition as defined in the claims for use in a method for treating, ameliorating, or preventing diseases caused by drug-resistant bacteria in a target, wherein the target is allowed to ingest the above-mentioned antibacterial composition or pharmaceutical composition, or the above-mentioned intestinal bacteria as an active ingredient thereof (hereinafter also collectively referred to as "the pharmaceutical composition of the present invention or the active ingredient thereof").

In the present invention, the "drug-resistant bacterium" means a bacterium that has resistance to antibacterial agents (such as antibiotics) and the drugs are ineffective or difficult to be effective. Further, the drug may be one drug or multiple drugs. That is, the drug-resistant bacteria according to the present invention also include multidrug-resistant bacteria. Such bacteria are not particularly limited, but examples thereof include carbapenem-resistant Enterobacteriaceae (CRE, KPC-2-producing Klebsiella pneumoniae, and the like), vancomycin-resistant enterococci (VRE, bacteria with vancomycin-resistant gene (vanA), and the like), Clostridium difficile, and Campylobacter jejuni. More specific examples include Klebsiella pneumoniae (ATCC BAA-1705), Enterococcus faecium (Orla-Jensen) Schleifer and Kilpper-Balz (ATCC 700221), Clostridioides difficile (Prevot) Lawson et al. (ATCC 43255, bacterial strain designation: VPI 10463), Clostridioides difficile (Prevot) Lawson et al. (ATCC BAA-1382, bacterial strain designation: 630), and Campylobacter jejuni 81-176 (ATCC BAA2151).

The "diseases caused by drug-resistant bacteria" include infectious diseases caused by drug-resistant bacteria. They also include diseases associated with the infection. Examples of such diseases include respiratory infectious diseases such as sepsis, peritonitis, meningitis, gastroenteritis, and pneumonia, urinary tract infectious diseases, surgical site infectious diseases, soft tissue infectious diseases, and medical device-related infectious diseases (such as medical device-related bloodstream infectious diseases).

In the present invention, the "pro-inflammatory bacterium" means a bacterium that induces inflammation in the intestinal tract, and examples thereof include adherent-invasive Escherichia coli (AIEC). More specifically, AIEC LF82 can be mentioned.

The "diseases caused by pro-inflammatory bacteria" include diseases caused or involved in inflammation caused by the bacteria. Examples of such diseases include inflammatory bowel diseases (including chronic inflammatory bowel diseases such as Crohn's disease, ulcerative colitis, and inflammatory bowel disease).

The pharmaceutical composition of the present invention or the active ingredient and the like thereof can be used for animals including humans, but there are no particular restrictions on animals other than humans, and various domestic animals, poultry, pets, laboratory animals, and the like can be targeted.

In addition, the ingestion target of the intestinal bacterium of the present invention includes animals carrying the drug-resistant bacteria and the like, regardless of the onset of diseases caused by the drug-resistant bacteria and the like. From the viewpoint of prevention, an animal that does not possess the bacteria or is suspected of possessing them may be allowed to ingest the pharmaceutical composition of the present invention or the active ingredient thereof.

The method for ingesting the pharmaceutical composition of the present invention or the active ingredient thereof is not particularly limited, and may be oral administration or parenteral administration (for example, administration into the intestinal tract), and in the case of oral administration, from the viewpoint of further improving the effects of the pharmaceutical composition of the present invention or the active ingredient thereof, it is preferable that the ingestion target of the pharmaceutical composition of the present invention or the active ingredient thereof has reduced production of gastric acid by ingestion of a proton pump inhibitor (PPI).

In addition, in the case of ingesting the pharmaceutical composition of the present invention or the active ingredient thereof, the amount ingested can be appropriately selected by those skilled in the art according to the target's age, weight, disease symptoms, health conditions, composition type (such as pharmaceutical product, and food and drink), ingestion method.

Preferable embodiments of the antibacterial composition and the pharmaceutical composition as well as the therapeutic use of the present invention have been described above, but the present invention is not limited to the above embodiments.

As shown in Examples described later, regarding the ability to suppress intestinal colonization possessed by the Th1 cell-inducible bacterium Klebsiella 2H7 strain (Kp2H7), the present inventors have succeeded in selecting 18 strains capable of exerting the same level of suppression ability as the 37 strains of intestinal bacteria derived from healthy subject #F. Therefore, also disclosed is an antibacterial composition against Th1 cell-inducible bacteria, containing an intestinal bacterium as an active ingredient, wherein the intestinal bacterium is at least one bacterium which includes a DNA composed of a base sequence set forth in any of SEQ ID NOs: 69, 80, 85 to 92, 94, 96, 98 to 101, 103, and 105 or a base sequence having at least 90% identity to the base sequence; the antibacterial composition which is a pharmaceutical composition; the antibacterial composition which is a pharmaceutical composition for treating, ameliorating, or preventing diseases caused by Th1 cells. Further disclosed is a method for suppressing proliferation or activation of Th1 cells in a target, a method for suppressing immunity in the target, or a method for treating, ameliorating, or preventing diseases caused by Th1 cells in the target, including allowing the target to ingest the antibacterial composition or at least one bacterium which includes a DNA composed of a base sequence set forth in any of SEQ ID NOs: 69, 80, 85 to 92, 94, 96, 98 to 101, 103, and 105 or a base sequence having at least 90% identity to the base sequence.

The "Th1 cell-inducible bacterium" is a bacterium that normally exists in the human oral cavity, and induces the proliferation or activation of Th1 cells by colonizing in the intestinal tract. It is preferably a bacterium that belongs to Klebsiella, more preferably belonging to Klebsiella pneumoniae or Klebsiella aeromobilis, and induces the proliferation or activation of Th1 cells in the intestinal tract. Further, the "Th1 cell-inducible bacterium" is preferably a bacterium that easily colonizes in an intestinal environment in which the diversity has changed as compared with the healthy state due to the administration of an antibacterial agent. It is also a bacterium that easily colonizes in an intestinal environment in which the diversity has changed as compared with the healthy state due to colitis or the like.

For an example of the "Th1 cell-inducible bacterium, " PTL 1 can be referred to, and typical examples include Kp2H7 strain, Ka11E12 strain, 34E1 strain, BAA-1705 strain, 700603 strain, and 40B3 strain belonging to Klebsiella. Among these, the Kp2H7 strain or the Ka11E12 strain is more preferable, and the Kp2H7 strain is particularly preferable. See Table 6 for details of these bacteria.

**[Table 6]**

| Bacterial Name | Supplier | Information from Supplier | Registry number |
|---|---|---|---|
| KCTC2242 | KCTC | http://kctc.kribb.re.kr/English/_ SearchView.aspx?sn=2242 | NCBI Taxonomy ID: 1049565 |
| 2552 | ATCC | https://www.atcc.org/Products /All/BAA-2552.aspx | NCBI Taxonomy ID: 507522 |
| KP-1 | - | - | NCBI Taxonomy ID: 1365186 |
| 700721 | ATCC | https://www.atcc.org/Products /All/700721.aspx | NCBI Taxonomy ID: 272620 |
| 13882 | JCM | https://www.atcc.org/Products /All/13882.aspx | NCBI Taxonomy ID: 1913574 |
| 40B3 | - | - | SAMD00083913 |
| 34E1 | - | - | SAMD00083911 |
| 1705 | ATCC | https://www.atcc.org/Products /All/BAA-1705.aspx | NCBI Taxonomy ID: 1276652 |
| 11E12 | - | - | SAMD00083912 |
| 700603 | ATCC | https://www.atcc.org/Products /All/700603.aspx | NCBI Taxonomy ID: 1276653 |
| 2 H7 | - | - | SAMD00083910 |

In addition, examples of the "Th1 cell-inducible bacterium" include a bacterium which contains a DNA composed of a nucleotide sequence having 90% or more (91% or more, 92% or more, 93% or more, 94% or more, 95% or more, 96% or more, 97% or more, 98% or more, and 99% or more) identity to the nucleotide sequence encoding the 16S rRNA of Kp2H7 strain, Ka11E12 strain, 34E1 strain, BAA-1705 strain, 700603 strain, or 40B3 strain, and also include a bacterium which contains a DNA composed of a nucleotide sequence having 70% or more (preferably 80% or more, more preferably 85% or more, further preferably 90% or more, and more preferably 94% or more (for example, 95% or more, 96% or more, 97% or more, 98% or more, and 99% or more)) homology or identity to the nucleotide sequence specific to Kp2H7 strain, Ka11E12 strain, 34E1 strain, BAA-1705 strain, 700603 strain, or 40B3 strain.

The "Th1 cell" is a subgroup of CD4 positive helper T-cells (Th cells) and means a cell that enhances cell-mediated immunity. In addition, the meaning of "activation of Th1 cells" includes the production of Th1 cytokines (such as IFN-γ) by the cells, induction of macrophages by the cytokines, activation of cells such as cytotoxic T-cells (CTL), and enhancement of cell-mediated immunity by the activation. Further, the meaning of "induction of the proliferation or activation of Th1 cells" also includes induction of differentiation from naive T-cells to Th1 cells which leads to proliferation or activation of Th1 cells.

The effect of inducing proliferation or activation of Th1 cells in the intestinal tract can be evaluated by quantitatively detecting Th1 cell-specific markers (such as CD4 and IFN-γ). Such quantitative detection can be performed by a known method, and can be performed by methods for detection using antibodies (immunological methods) such as flow cytometry, imaging cytometry, ELISA method, radioimmunoassay, immunohistochemical staining, immunoprecipitation, immunoblotting, and antibody array analysis.

Regarding whether or not any bacterium has an effect of inducing proliferation or activation of Th1 cells in the intestinal tract, for example, it can be determined that the bacterium has an effect of inducing proliferation or activation of Th1 cells in the intestinal tract when the proportion of IFN-γ⁺ cells in CD4⁺TCRβ⁺ T-cells in the intestinal tract detected by flow cytometry is 10% or more (it is preferable to determine, when the proportion is 25% or more, that the bacterium has an effect of inducing proliferation or activation of Th1 cells in the intestinal tract, and it is more preferable to determine, when the proportion is 30% or more, that the bacterium, substance, has an effect of inducing proliferation or activation of Th1 cells in the intestinal tract).

The "diseases caused by Th1 cells" mean diseases induced by proliferation or activation of Th1 cells, and examples thereof include inflammatory bowel diseases (including chronic inflammatory bowel diseases such as Crohn's disease, ulcerative colitis, and inflammatory bowel disease), type 1 diabetes, rheumatoid arthritis, experimental autoimmune encephalomyelitis (EAE), multiple sclerosis, autoimmune diseases such as systemic lupus erythematosus, and chronic inflammatory diseases. In addition, the "immunity" suppressed in the present invention includes not only mucosal immunity (such as intestinal immunity) but also systemic immunity. Furthermore, not only cell-mediated immunity but also humoral immunity is included.

In addition, when the antibacterial composition is used as a pharmaceutical composition, it may further contain known substances (such as anti-inflammatory agents and immunosuppressants) used for the treatment, prevention, or amelioration of diseases caused by Th1 cells, or may be used in combination with such substances.

### <Composition for Examining Diseases Caused by Drug-Resistant Bacteria>

In the present invention, the presence of intestinal bacteria that can suppress the colonization of drug-resistant bacteria in the intestinal tract has been clarified. Therefore, by detecting the presence of the intestinal bacteria, it becomes possible to examine diseases caused by drug-resistant bacteria

Therefore, also disclosed is a composition for examining diseases caused by the following drug-resistant bacteria.

A composition for examining diseases caused by drug-resistant bacteria and the like, containing an antibody that specifically recognizes the intestinal bacterium.

A composition for examining diseases caused by drug-resistant bacteria, containing a polynucleotide for detecting a nucleotide sequence specific to the drug-resistant bacteria.

The "antibody that specifically recognizes the intestinal bacterium" be a polyclonal antibody, a monoclonal antibody, or a functional fragment of antibody (for example, Fab, Fab', F(ab')2, variable region fragment (Fv), disulfide bond Fv, single chain Fv (scFv), sc(Fv)2, diabody, multispecific antibody, or a polymer thereof) as long as it can specifically recognize the bacterium. If the antibody is a polyclonal antibody, it can be obtained by immunizing a host animal with an antigen (peptide, polynucleotide, sugar chain, lipid, derived from the intestinal bacterium) and purifying it from its antiserum by a conventional means (such as salting-out, centrifugation, dialysis, or column chromatography). In addition, the monoclonal antibody can be produced by a hybridoma method or a recombinant DNA method.

Further, as the antibody used for the examination, it is possible to use an antibody bound with a labeling substance. By detecting the labeling substance, it is possible to directly measure the amount of antibody bound to the intestinal bacterium or a substance derived from the bacterium. The labeling substance is not particularly limited as long as it can bind to an antibody and can be detected by a chemical or optical method, and examples thereof include fluorescent dyes (such as GFP), enzymes (such as HRP), and radioactive substances.

In addition to the antibody component, the examination composition may contain additional components acceptable as a composition. Examples of the additional components include carriers, excipients, disintegrants, buffer agents, emulsifiers, suspensions, stabilizers, preservatives, antiseptics, saline, labeling substances, and secondary antibodies. In addition to the above examination compositions, it is possible to combine substrates required for detection of labeling substances, positive controls and negative controls, buffer solutions used for diluting or washing the sample, tubes or plates used for the reaction of the sample with the antibody, or use as an examination kit for diseases caused by drug-resistant bacteria is possible. In addition, when an unlabeled antibody is used as an antibody standard sample, it is possible to combine a labeled substance (such as secondary antibody, protein G, or protein A) that binds to the antibody. Further, the kit for examining diseases caused by drug-resistant bacteria can include an instruction manual for use of the kit.

Further, the examination composition can be combined with an apparatus for detecting the antibody. Examples of the apparatus include a flow cytometry apparatus and a microplate reader.

The "polynucleotide for detecting a nucleotide sequence specific to the intestinal bacterium" is not particularly limited as long as the sequence specific to the bacterium is detected, and examples thereof include polynucleotides according to any of the following (a) and (b), each having a chain length of at least 15 nucleotides.
(a) A polynucleotide that is a pair of primers designed to sandwich the specific nucleotide sequence, and
(b) A polynucleotide that is a primer or probe that hybridizes to a nucleotide sequence containing the specific nucleotide sequence.

The polynucleotide has a base sequence complementary to the nucleotide sequence of the intestinal bacterium.

Here, "complementary" does not have to be perfectly complementary as long as it hybridizes. These polynucleotides usually have 80% or more, preferably 90% or more, more preferably 95% or more, and particularly preferably 100% homology to the nucleotide sequence.

The "chain length" of the polynucleotide of the present invention, when used as a primer, is usually 15 to 100 nucleotides, preferably 17 to 30 nucleotides, and more preferably 20 to 25 nucleotides. In addition, when used as a probe, it is usually 15 to 1000 nucleotides, and preferably 20 to 100 nucleotides.

The polynucleotide may be a DNA or RNA, or may have its nucleotide replaced with an artificial nucleic acid such as LNA (registered trademark, crosslinked nucleic acid), ENA (registered trademark, 2'-O, 4'-C-Ethylene-bridged nucleic acids), GNA (glycerol nucleic acid), TNA (threose nucleic acid), or PNA (peptide nucleic acid) in part or all of it.

Note that the polynucleotide can be chemically synthesized using a commercially available nucleotide automatic synthesizer. Further, as the polynucleotide used for the examination of the present invention, it is possible to use a polynucleotide bound with a labeling substance. The labeling substance is not particularly limited as long as it can bind to a polynucleotide and can be detected by a chemical or optical method, and examples thereof include fluorescent dyes (such as DEAC, FITC, R6G, TexRed, and Cy5) as well as dyes such as DAB (chromogen), enzymes, and radioactive substances in addition to fluorescent dyes.

In addition to the above-mentioned polynucleotide, the examination composition may contain additional pharmacologically acceptable components. Examples of the additional components include buffer agents, emulsifiers, suspensions, stabilizers, antiseptics, and saline.

In addition to the above examination compositions, it is possible to combine standard samples such as substrates required for detection of labeling substances added to the polynucleotide, positive controls and negative controls, and buffer solutions used for diluting or washing the sample, thereby combining tubes or plates used for the reaction of the sample with the polynucleotide, or use as an examination kit for diseases caused by drug-resistant bacteria is possible. Moreover, the kit for examining diseases caused by drug-resistant bacteria and the like can include an instruction manual for use of the kit.

In addition, the examination composition can be combined with an apparatus for detecting a nucleotide sequence specific to the intestinal bacterium. Examples of the apparatus include a thermal cycler, a sequencer, and a microarray.

Furthermore, also disclosed is a method for examining diseases caused by drug-resistant bacteria using the above-mentioned antibody, polynucleotide, or examination composition. Specifically, disclosed is
a method for examining diseases caused by drug-resistant bacteria, including the steps of contacting the antibody, polynucleotide, or examination composition with a sample isolated from a subject, and detecting the presence or absence of an intestinal bacterium in the intestinal tract by the contact.

The subject is not particularly limited, and examples thereof include animals such as humans suspected of suffering from a disease caused by drug-resistant bacteria. Further, the sample isolated from the subject is not particularly limited, but a fecal sample of a subject, a culture thereof, or a polypeptide, polynucleotide, sugar chain, lipid or the like extracted therefrom is preferably used in the method

As the method for detecting the presence or absence of an intestinal bacterium by contacting the sample with the antibody or an examination composition containing the same, examples thereof include methods for detection using antibodies (immunological methods) such as ELISA method, immunoblotting, antibody array analysis, immunohistochemical staining, flow cytometry, imaging cytometry, radioimmunoassay, and immunoprecipitation.

Further, as the method for detecting the presence or absence of an intestinal bacterium by contacting the sample with the polynucleotide or an examination composition containing the same, it is possible to use PCR (RT-PCR, real-time PCR, and quantitative PCR), DNA microarray analysis, Northern blotting, 16s rRNA sequencing, next-generation sequencing (synthetic sequencing (sequencing-by-synthesis, for example, sequencing by Solexa Genome Analyzer or Hiseq (registered trademark) 2000 manufactured by Illumina), pyrosequencing (for example, sequencing by a sequencer GSLX or FLX manufactured by Roche Diagnostics (454) (so-called 454 sequencing)), and ligase reaction sequencing (for example, sequencing by SoliD (registered trademark) or 5500xl manufactured by Life Technologies)), bead array method, in situ hybridization, dot blotting, RNase protection assay, mass spectrometry, genomic PCR method, and Southern blotting.

The "examination" of a disease caused by drug-resistant bacteria includes not only the presence or absence of the onset of the disease but also the risk of the onset of the disease, and if the presence of an intestinal bacterium is detected in the intestinal tract by the above-mentioned method, it can be determined that a disease caused by drug-resistant bacteria has not developed or the risk of developing the disease is low.

Diagnosis of a disease caused by drug-resistant bacteria in a subject is usually made by a doctor (including a person who has been instructed by a doctor), but the data obtained by the method is useful for diagnosis by a doctor. Therefore, the method can also be expressed as a method of collecting and presenting data useful for diagnosis by a doctor.

Further, disclosed is a companion diagnostic method using the above-mentioned examination method and a drug thereof. That is, also disclosed is the following.

A method for determining the effectiveness of the pharmaceutical composition or the active ingredient thereof in the treatment, amelioration, or prevention of diseases caused by drug-resistant bacteria, including the steps of contacting the antibody, polynucleotide, or examination composition with a sample isolated from a subject, and detecting the presence or absence of the intestinal bacteria by the contact, wherein in the step, when the presence of the bacteria is not detected, it is determined that the pharmaceutical composition or the active ingredient thereof in the subject is highly effective in treating, ameliorating, or preventing the disease.

A method for treating, ameliorating, or preventing diseases caused by drug-resistant bacteria, including a step of allowing a patient determined by the determination method to have high effectiveness of the pharmaceutical composition or the active ingredient thereof to ingest the pharmaceutical composition or the active ingredient thereof.

A composition for treating, ameliorating, or preventing diseases caused by drug-resistant bacteria, containing the intestinal bacterium as an active ingredient, wherein the composition is ingested by a subject determined to have high effectiveness by the determination method.

### <Method for Screening Intestinal Bacteria Having Antibacterial Activity against Drug-Resistant Bacteria in Intestinal Tract>

It has also been clarified for the first time by the present inventors that among intestinal bacteria, there are bacteria that suppress the colonization of drug-resistant bacteria in the intestinal tract. Therefore, disclosed is a method for screening intestinal bacteria having antibacterial activity against drug-resistant bacteria, including the following steps.

A step of allowing a non-human germ-free animal to ingest drug-resistant bacteria and the like and test intestinal bacteria in the intestinal tract,
a step of detecting the drug-resistant bacteria in the intestinal tract of the non-human germ-free animal, and
a step of determining the test intestinal bacteria as intestinal bacteria having antibacterial activity against the drug-resistant bacteria when the number of bacteria detected in the step is reduced as compared with the case where the test intestinal bacteria are not ingested.

The "drug-resistant bacteria" are as described above. The "non-human germ-free animal" means a non-human animal that is born and grown under germ-free conditions. Examples of non-human animals include, but are not limited to, mice, rats, monkeys, pigs, cows, horses, sheep, goats, chickens, ducks, ostriches, domestic ducks, dogs, cats, rabbits, and hamsters. Moreover, in these animals, mice are preferably used.

The test intestinal bacteria to be ingested by non-human germ-free animals may be any bacteria present in the intestines of animals, examples of such animals include humans and non-human animals (such as mice, rats, monkeys, pigs, cows, horses, sheep, goats, chickens, ducks, ostriches, domestic ducks, dogs, cats, rabbits, and hamsters). In addition, the test intestinal bacteria to be ingested by non-human germ-free animals may be isolated intestinal bacteria, and examples thereof include a sample containing intestinal bacteria (for example, a fecal sample of the animal or a culture thereof).

Further, the method for "ingesting" the test intestinal bacteria and drug-resistant bacteria to a non-human animal is not particularly limited, and is usually performed by oral administration, but may be parenteral administration (for example, administration into the intestinal tract). In addition, the test intestinal bacteria and drug-resistant bacteria may be ingested at the same time, the drug-resistant bacteria may be ingested by the non-human animal after the test intestinal bacteria are ingested by the animal, or the test intestinal bacteria may be ingested by the non-human animal after the drug-resistant bacteria are ingested by the animal.

The "detection" of drug-resistant bacteria in the intestinal tract can be performed by detecting a nucleotide sequence specific to the drug-resistant bacteria. Examples of the detection method include PCR (RT-PCR, real-time PCR, and quantitative PCR), DNA microarray analysis, Northern blotting, 16s rRNA sequencing, next-generation sequencing (synthetic sequencing (sequencing-by-synthesis, for example, sequencing by Solexa Genome Analyzer or Hiseq (registered trademark) 2000 manufactured by Illumina), pyrosequencing (for example, sequencing by a sequencer GSLX or FLX manufactured by Roche Diagnostics (454) (so-called 454 sequencing)), and ligase reaction sequencing (for example, sequencing by SoliD (registered trademark) or 5500xl manufactured by Life Technologies)), bead array method, in situ hybridization, dot blotting, RNase protection assay, mass spectrometry, genomic PCR method, and Southern blotting.

Further, the "detection" of drug-resistant bacteria in the intestinal tract can be performed, for example, by detecting an amino acid sequence specific to the drug-resistant bacteria. Examples of the detection method include methods for detection using antibodies (immunological methods) such as ELISA method, immunoblotting, antibody array analysis, immunohistochemical staining, flow cytometry, imaging cytometry, radioimmunoassay, and immunoprecipitation. The timing of detection is not particularly limited, and those skilled in the art can appropriately adjust the timing according to the type of animal to be used.

Note that if the screening method fails to select intestinal bacteria having antibacterial activity against drug-resistant bacteria in a single trial, an intestinal sample containing the obtained bacteria is ingested by a new non-human germ-free animal as the next test intestinal bacteria, and the above-mentioned screening is performed multiple times, making it possible to isolate the intestinal bacteria having the antibacterial activity.

### [Examples]

### (Example 1)

As shown in the upper part of Fig. 1, germ-free mice were administered with Klebsiella 2H7 strains (Kp2H7), and one week later, fecal samples of healthy volunteers were administered.

Specifically, the germ-free mice used were C57BL/6N at 4 to 8 weeks of age (CLEA Japan, Inc.), which were kept in a rearing vinyl isolator (germ-free isolator) (manufactured by ICM Co., Ltd.; ICM-1B) under the conditions of free-drinking water and feeding for one week or longer to acclimate them to the environment. The age at the start of the experiment was 8 to 14 weeks of age. The same applies to the other Examples in the present specification.

A bacterial solution of Klebsiella was placed in an LB liquid medium and cultured overnight at 37°C to adjust the OD value to 1.2 (equivalent to 1*10^9 CFU/mL), and 200 µL/individual (equivalent to 2*10^8 CFU/individual) of bacterial solution was administered into the stomach of mice using a sonde.

For the fecal samples, feces provided by Japanese healthy subject volunteers (#A, #F, #I, #J, and #K) were diluted 5-fold by weight with a glycerol PBS solution (final concentration of glycerol: 20% by volume), which was filtered through a 100 µm diameter filter, and the resultant was stored as a stock solution at -80°C. At the time of fecal administration, the stock solution was diluted 10-fold with PBS in an anaerobic chamber, and administered in an amount of 200 µL/individual into the stomach of each mouse using a sonde.

Mouse fecal samples were dissolved in a solution of glycerol (final concentration 20%) and EDTA (final concentration 10 mM) mixed in PBS at a proportion of 50 mg feces/mL. The fecal lysate was seeded after appropriate dilution in DHL medium containing 50 mg/L ampicillin and 50 mg/L spectinomycin, and after culturing at 37°C overnight, the number of colonies was counted and the number of CFUs per 1 g of feces was calculated.

In the other Examples in the present specification, bacterial solution of Klebsiella and feces were administered in the same manner, and CFUs were counted in the same manner.

As a result, as shown in the lower part (graph) of Fig. 1, five types of feces were used, and a significant decrease in the bacterial amount of Kp2H7 was observed in all the samples.

### (Example 2) Isolation of Bacteria from Feces of Healthy Subject Volunteers

Each of the frozen fecal samples derived from feces of healthy subjects F, K, and I (feces F, feces K, and feces I) prepared in Example 1 was thawed at room temperature, diluted with PBS, and cultured in an agar plate of EG medium, modified GAM agar medium (Nissui Pharmaceutical Co., Ltd.; 05426), REINFORCED CLOSTRIDIAL AGAR (RCM AGAR) (Thermo Fisher Scientific Inc; CM0151), or Schaedler blood medium (manufactured by Wako; 517-45805) under an anaerobic environment of at 37°C and 10% CO₂, and the colonies formed were isolated. From feces F, 37 strains were isolated, 42 strains were isolated from feces I, and 47 strains were isolated from feces K. After that, the feces K was isolated again, and finally 68 strains were isolated from the feces K.

For the isolated bacteria, the gene sequence was analyzed and the bacterial species was estimated by 16S rDNA analysis by the Sanger method. Sequence analysis was performed using 3130 DNA Analyzer manufactured by Thermo Fisher Scientific and a primer set having the following sequences.
27 Forward-mod: 5'-AGRGTTTGATYMTGGCTCAG-3' (SEQ ID NO: 148)
1492 Reverse: 5'-GGYTACCTTGTTACGACTT-3' (SEQ ID NO: 149)

Here, R: A or G, Y: C or T, and M: A or C.

Furthermore, the genome sequences of the 37 bacterial strains derived from feces F were determined using a next-generation sequencer. That is, genome sequencing was performed using MiSeq manufactured by Illumina and Sequel manufactured by Pacific Biosciences, and all genome sequences were obtained by hybrid assembly using Unicycler. By extracting the 16S rRNA sequence using RNAmmer for each of these genome sequences, a more accurate sequence was obtained, including both terminal sequences that could not be determined by the 16S rDNA sequence determined by the Sanger method.

Tables 1 to 4 show the results of this analysis. In addition, Fig. 2 shows the results of 16S meta-analysis of three types of feces derived from donors F, I, and K.

### (Example 3)

As shown in the upper part of Fig. 3, Kp2H7 was administered to germ-free mice, and one week later, mixed isolated bacteria (37 strains derived from feces F (F37mix), 42 strains derived from feces I (I42mix), and 47 strains derived from feces K (K47mix)) or feces I were administered.

The isolated bacteria were cultured in mGAM liquid medium, EG medium, or CM0149 medium at 37°C in an anaerobic chamber for 24 to 48 hours, and mixed. The mixed solution was concentrated 5 times, and 200 µL/individual (equivalent to total bacterial amount 1*10^9 CFU/animal) of bacterial solution was administered into the stomach using a sonde. The mixed isolated bacterial strains in the following Examples were also administered in the same manner.

As a result, as shown in the lower part (graph) of Fig. 3, it was clarified that the 37 strains derived from feces F had the same activity as feces I in terms of the ability to eliminate Klebsiella from the mouse intestinal tract.

### (Example 4)

As shown in the upper part of Fig. 4, Kp2H7 was administered to germ-free mice, and one week later, the mixed isolated bacteria (37 strains derived from feces F and 68 strains derived from feces K) were administered. As a result, as shown in the lower part (graph) of Fig. 4, 37 strains derived from feces F and 68 strains derived from feces K equally eliminated Klebsiella from the mouse intestinal tract.

### (Example 5)

As shown in the upper part of Fig. 5, Kp2H7 was administered to germ-free mice, and one week later, mixed isolated bacteria (F37mix) were administered, and 200 mg/L of ampicillin was administered by drinking water after the administration of the isolated bacteria. In order to investigate the change in the bacterial amount of each of the administered bacteria, PCR was performed using a specific primer of each of the bacteria. Table 7 shows the primers used in the analysis.

**[Table 7]**

| | Forward Primer Name | SEQ ID NO: | Reverse Primer Name | SEQ ID NO: |
|---|---|---|---|---|
| f01 | f01_42H6_Fw1 | 150 | f01_42H6_Rv1 | 151 |
| f02 | f02_4218_Fw1 | 152 | f02_4218_Rv3 | 153 |
| f05 | f05_43D4_Fw4 | 154 | f05_43D4_Rv2 | 155 |
| f06 | f06_42F4_Fw1 | 156 | f06_42F4_Rv1 | 157 |
| f07 | f07_42J6_Fw1 | 158 | f07_42J6_Rv1 | 159 |
| f08 | f08_42B5_Fw1 | 160 | f08_42B5_Rv1 | 161 |
| f09 | f09_43F1_Fw1 | 162 | f09_43F1_Rv1 | 163 |
| f10 | f10_43J3_Fw1 | 164 | f10_43J3_Rv1 | 165 |
| f12 | f12_42H4_Fw1 | 166 | f12_42H4_Rv1 | 167 |
| f13 | f13_42H8_Fw1 | 168 | f13_42H8_Rv1 | 169 |
| f14 | f14_42L4_Fw1 | 170 | f14_42L4_Rv1 | 171 |
| f16 | f16_43M1_Fw2 | 172 | f16_43M1_Rv2 | 173 |
| f17 | f17_42I7_Fw3 | 174 | f17_42I7_Rv3 | 175 |
| f18 | f18_42I2_Fw5 | 176 | f18_42I2_Rv5 | 177 |
| f19 | f19_43G2_Fw2 | 178 | f19_43G2_Rv2 | 179 |
| f20 | f20_43G1_Fw2 | 180 | f20_43G1_Rv2 | 181 |
| f21 | f21_42A8_Fw2 | 182 | f21_42A8_Rv6 | 183 |
| f22 | f22_43C3_Fw2 | 184 | f22_43C3_Rv2 | 185 |
| f24 | f24_42I4_Fw1 | 186 | f24_42I4_Rv1 | 187 |
| f25 | f25_42J1_Fw2 | 188 | f25_42J1_Rv2 | 189 |
| f26 | f26_42K2_Fw1 | 190 | f26_42K2_Rv1 | 191 |
| f27 | f27_42J5_Fw2 | 192 | f27_42J5_Rv2 | 193 |
| f28 | f28_43A3_Fw1 | 194 | f28_43A3_Rv1 | 195 |
| f29 | f29_43J8_Fw2 | 196 | f29_43J8_Rv2 | 197 |
| f30 | f30_43A5_Fw1 | 198 | f30_43A5_Rv1 | 199 |
| f31 | f31_43J5_Fw1 | 200 | f31_43J5_Rv2 | 201 |
| f32 | f32_42A7_Fw1 | 202 | f32_42A7_Rv2 | 203 |
| f33 | f33_43N2_Fw1 | 204 | f33_43N2_Rv1 | 205 |
| f34 | f34_43K4_Fw1 | 206 | f34_43K4_Rv1 | 207 |
| f35 | f35_42L8_Fw2 | 208 | f35_42L8_Rv2 | 209 |
| f37 | f37_42G1_Fw3 | 210 | f37_42G1_Rv4 | 211 |

As a result, as shown in the lower part of Fig. 5, the bacterial amount of Klebsiella was transiently increased by the administration of ampicillin, but decreased again thereafter.

In addition, the change over time of the abundance ratio of each isolated bacterium in the total bacterial amount was analyzed together with that of Kp2H7. Figs. 6A to 6H show the results obtained. Furthermore, the bacterial amount of Klebsiella and the Spearman's rank correlation coefficient of each bacterium were calculated and arranged in order of high positive correlation. Fig. 7 shows the results obtained.

As shown in Fig. 7, it was found that the strains belonging to Bacteroidetes moved regardless of the movement of Klebsiella. On the other hand, many of the strains having a negative correlation belonged to the genus Furmicutes.

### (Example 6)

As shown in Fig. 9, 37 the strains derived from feces F were divided into 8 strains belonging to Bacteroidetes (F8mix) and the 29 other bacterial strains (F29mix), and mixed respectively, and after Kp2H7 was colonized in germ-free mice, the mixed isolated bacteria were administered. Note that the phylogenetic trees shown in Figs. 9, 10, and 12 were prepared by the Neighbor-joining method using MEGA X with the DNA base sequence of the 16S rDNA analysis result of the isolated bacteria by the Sanger method.

As a result, as shown in Fig. 8, F37mix and F29mix equally eliminated Klebsiella from the mouse intestinal tract. On the other hand, in the F8mix administration group belonging to Bacteroidetes, the bacterial amount of Klebsiella did not change, suggesting that F8mix is not related to the elimination of Klebsiella.

### (Example 7)

From the 37 strains, 18 strains were selected by excluding strains belonging to Bacteroidetes, strains duplicate at the 16S rDNA level, strains disappeared by ampicillin administration, and strains showing behavior unrelated to Klebsiella (see Fig. 10).

Then, as shown in the upper part of Fig. 11, the Kp2H7 strains were administered to germ-free mice, and one week later, mixed isolated bacteria (37 strains derived from feces F (F37mix), 18 strains (Fl8mix), and 42 strains derived from feces I (I42mix)) were administered.

As a result, as shown in the lower part (graph) of Fig. 11, it was clarified that the 18 strains shown in Fig. 10 was also able to exhibit the ability to eliminate Klebsiella strains as much as the 37 strains.

### (Example 8)

The 18 strains shown in Fig. 10 were divided into 4 groups based on the phylogenetic tree (Blautia, Lachonoclostridum, other Firmicutes, and other Phyla) (see Fig. 12). Furthermore, these 4 groups were subtracted from the 18 bacterial strains (F18mix) to prepare bacterial strain groups of F15mix (Fl8mix-other phyla), F12mix (Fl8mix-Lachnoclostridium), F14mix (Fl8mix-Blautia), F13mix (Fl8mix-other Firmicutes). In addition, a bacterial strain set was also prepared by mixing 13 strains (Fl3mix (F31-18mix)), which were obtained by removing the duplicated strains from the 37 strains and further removing the above-described 18 strains from the remaining 31 strains. Then, as shown in the upper part of Fig. 13, Kp2H7 was administered to germ-free mice, and one week later, each isolated bacterium mixed as described above was administered.

As a result, as shown in the lower part (graph) of Fig. 13, F18mix best eliminated Klebsiella. However, the bacterial amount of Klebsiella significantly increased regardless of which group was excluded. In addition, as shown in Fig. 14, also in terms of the CFU of fecal Klebsiella in each administration group at the time of day 28 in the experiment shown in Fig. 13, F18mix was able to reduce Klebsiella statistically significantly by more than any other group excluding F37mix.

From the above, it was suggested that all of the four groups shown in Fig. 10 were involved in the elimination of Klebsiella, and eliminated Klebsiella as a community.

Further, in the experiment shown in Fig. 13, lymphocytes in the lamina propria of the large intestine were extracted from mice in the F37mix, F18mix, and F31-18mix groups and subjected to analysis by flow cytometry.

As a result, as shown in Fig. 15, it was clarified that the proportion of CD4+IFNγ+ cells was high in the F13mix (F31-18mix) group, and that the induction of Thl cells was suppressed in F37mix and F18mix.

### (Example 9)

As shown in Example 8, in the experiment of removing the bacteria of each group from F18mix, F15mix obtained by removing the 3 strains of other Phyla had the lowest ability to eliminate Klebsiella. Therefore, paying attention to this group, the present inventors prepared groups each obtained by removing any one of these 3 strains (E. coli, Bifidobacterium, and Fusobacterium) from the 18 strains derived from feces F. Then, as shown in the upper part of Fig. 16, the Kp2H7 strains were administered to germ-free mice, and one week later, isolated bacteria mixed as described above, F18mix, or F15mix were administered.

As a result, as shown in the lower part (graph) of Fig. 16, by removing any of the above three strains, an increase of about 1 log in the bacterial amount of Klebsiella was observed, suggesting that all of the strains were involved in the elimination of Klebsiella.

### (Example 10)

In order to explore the mechanism of eliminating Klebsiella of F37mix, the present inventors focused on whether host immunity was involved in this mechanism. Therefore, as shown in the upper part of Fig. 17, the Klebsiella 2H7 strain was administered to a germ-free Rag2^{-/-}γc^{-/-} mouse, MyD88^{-/-}Triff^{-/-} mouse, or wild-type mouse, and one week later, mixed F37mix was administered.

As a result, as shown in the lower part (graph) of Fig. 17, the same degree of elimination of the Klebsiella 2H7 strain by F37mix was observed in all types of mice. This suggests that the major innate immunity and acquired immunity of the host are not involved in the elimination of Klebsiella.

Next, the effect of eliminating the isolated bacterial strains on pathogens and drug-resistant bacteria other than the Kp2H7 strain was evaluated. Note that in the analysis, the primers shown in Table 8 were used as primers specific to the bacteria.

**[Table 8]**

| Target Bacteria and the Like | Primer Name | SEQ ID NO: |
|---|---|---|
| CRE | KPC-F684 | 212 |
| | KPC-R860 | 213 |
| VRE (vanA) | VanABF_JCM1998 | 214 |
| | VanAR_JCM1998 | 215 |
| ESBL | ESBL-F_AAC2001 | 216 |
| | ESBL-R_AAC2001 | 217 |
| LF82-specific pMT gene | LF82-F | 218 |
| | LF82-R | 219 |
| C. jejuni | C.jejumi_F | 220 |
| | C.jejuni_R | 221 |
| Universal | Universal _Fw_1387 | 222 |
| | Universal _Rv_1492 | 223 |
| C.diff | tpi-F | 224 |
| | tpi-R | 225 |
| C.upsaliensis | Cupsaliensis_2F | 226 |
| | Cmpsaliensis_2R | 227 |
| C.upsaliensis | Cupsaliensis_3F | 228 |
| | Cupsaliensis_3R | 229 |
| Kp-P1 | khe-Fw | 230 |
| | khe-RV | 231 |
| E.gallinarum | Eg_2F | 232 |
| | Eg_2R | 233 |
| P.mirabilis | P.mirabillis _NatMicro_F | 234 |
| | P.mirabillis _NatMicro_R | 235 |

### (Example 11)

First, as shown in the upper part of Fig. 18, carbapenem-resistant Klebsiella (CRE) was administered to germ-free mice, and one week later, mixed F37mix, K68mix, or I42mix was administered. The results obtained are shown at the lower part of Fig. 18.

Note that a bacterial solution of CRE was placed in an LB liquid medium and cultured overnight at 37°C to adjust the OD value to 1.2 (equivalent to 1*10^9 CFU/mL), and 200 µL/individual (equivalent to 2*10^8 CFU/individual) of bacterial solution was administered into the stomach of mice using a sonde. Regarding the CFU count for CRE, DHL medium containing 30 mg/L ampicillin and 30 mg/L spectinomycin was used as a selective medium and cultured overnight at 37°C under aerobic conditions.

In addition, the mice one month after administration of the mixed isolated bacteria were dissected, and the large intestine was fixed with 4% PFA and embedded in paraffin to prepare sliced sections. These sections were stained with hematoxylin solution and eosin solution to observe inflammatory images of the tissues. Fig. 19 shows the obtained results.

As shown in the lower part (graph) of Fig. 18, F37mix and K68mix showed equivalent CRE elimination ability, and I42mix was slightly inferior to them. In addition, as shown in Fig. 19, no inflammatory findings such as ulceration or infiltration of inflammatory cells were observed in any of the mice. From this, it was clarified that the induction of inflammation in the large intestine was suppressed by administering each of the isolated bacterial mixed groups.

### (Example 12)

As shown in the upper part of Fig. 20, vancomycin-resistant Enterococcus faecium (VRE) was administered to germ-free mice, and one week later, mixed F37mix, K68mix, or I42mix was administered.

Note that a bacterial solution of VRE was placed in an LB liquid medium and cultured overnight at 37°C to adjust the OD value to 1.2 (equivalent to 1*10^9 CFU/mL), and 200 µL/individual (equivalent to 2*10^8 CFU/individual) of bacterial solution was administered into the stomach of mice using a sonde. Regarding the CFU count for VRE, VRE medium (Nippon Becton) was used and cultured overnight at 37°C under aerobic conditions.

As a result, as shown in the lower part (graph) of Fig. 20, K68mix exhibited the highest elimination ability on VRE. In addition, as shown in Fig. 21, no inflammatory findings such as ulceration or infiltration of inflammatory cells were observed in any of the mice. From this, it was clarified that the induction of inflammation in the large intestine was suppressed by administering each of the isolated bacterial mixed groups.

### (Example 13)

As shown in the upper part of Fig. 22, adhesion-invasive E.coli (AIEC LF82) was administered to germ-free mice, and one week later, mixed F37mix, K68mix, and I42mix were administered.

Note that a bacterial solution of AIEC LF82 was placed in an LB liquid medium and cultured overnight at 37°C to adjust the OD to 1.2 (equivalent to 1*10^9 CFU/mL), and 200 µL/individual (equivalent to 2*10^8 CFU/individual) of bacterial solution was administered into the stomach of mice using a sonde. Regarding the CFU count for AIEC LF82, MacConkey medium containing 1 mg/L cefotaxime was used as a selective medium and cultured overnight at 37°C under aerobic conditions to calculate the CFU.

As a result, as shown in the lower part (graph) of Fig. 22, F37mix had the highest elimination ability on AIEC LF82.

### (Example 14)

As shown in the upper part of Fig. 23, ESBL-producing Klebsiella (Kp-ESBL) (ATCC 700721) was administered to germ-free mice, and one week later, mixed F37mix, K68mix, I42mix, or feces F was administered.

Note that a bacterial solution of Kp-ESBL was placed in an LB liquid medium and cultured overnight at 37°C to adjust the OD to 1.2 (equivalent to 1*10^9 CFU/mL), and 200 µL/individual (equivalent to 2*10^8 CFU/individual) of bacterial solution was administered into the stomach of mice using a sonde. Regarding the CFU count for Kp-ESBL, DHL medium containing 30 mg/L ampicillin and 30 mg/L spectinomycin was used as a selective medium and cultured overnight at 37°C under aerobic conditions for calculation.

As a result, as shown in the lower part (graph) of Fig. 23, F37mix and K68mix showed the same Kp-ESBL elimination ability as feces F.

### (Example 15)

As shown in the upper part of Figs. 24 and 25, Campylobacter jejuni 81-176 (ATCC BAA2151) was administered to germ-free mice, and one week later, mixed F37mix, K68mix, I42mix, or feces F was administered.

The bacterial solution of Campylobacter jejuni was placed in a TS liquid medium, placed in an anaerobic jar together with a slightly aerobic AnaeroPack, and cultured at 42°C for 48 hours, and the bacterial solution was administered into the stomachs of the mice using a sonde.

For Campylobacter jejuni, CFU and qPCR were used to show the bacterial amount.

CHROMagar Campylobacter was used for CFU counting, which was placed in an anaerobic jar together with a slightly aerobic AnaeroPack and cultured at 42°C for 48 hours. Fig. 24 shows the obtained results.

The qPCR measurement was performed according to the following procedure.

LightCycler (registered trademark) 480II (Roche; 05015243001) and Thunderbird (registered trademark) SYBR (registered trademark) qPCR Mix (TOYOBO; QPS-201X5) were used to amplify and quantify with Campylobacter jejuni genome-specific primers and universal bacterial primers, and the calculated DNA concentration ratio was used as the abundance ratio of Campylobacter jejuni. Fig. 25 shows the obtained results.

Note that the primer sets set forth in SEQ ID NOs: 220 and 221 were used as Campylobacter jejuni genome-specific primers for qPCR, and the primer sets set forth in SEQ ID NOs: 222 and 223 were used as universal bacterial primers.

In addition, the bacterial genome was extracted by the following steps.

A PBS solution containing EDTA and glycerol (final concentration of EDTA: 10 mM, and final concentration of glycerol: 20% by volume) was added 5 times by weight to 10 mg of mouse feces, and the mixture was crushed and suspended with vigorous shaking and stirring. Added to 100 µL of sample solution was 800 µL of 10 mM Tris/10 mM EDTA buffer solution (pH 8.0, also referred to as "TE10" hereinafter) having 15 mg of lysozyme (manufactured by Sigma-Aldrich, Lysozyme from chicken egg white; L4919) and 5 µL of RNase (manufactured by Thermo Fisher Scientific, PureLink RNase A (20 mg/mL); 12091-021) dissolved therein, and the mixture was shaken at 37°C for 1 hour. Subsequently, 2,000 U of Achromopeptidase (registered trademark) (Wako; 015-09951) was added, and the mixture was shaken at 37°C for 30 minutes for lysis. Then, 50 µL of a 20% SDS TE10 solution and 50 µL of a TE10 solution having Proteinase K (Roche, Proteinase K, recombinant, PCR Grade; 0311585.101) at a final concentration of 20 mg/ml dissolved therein were added, and the mixture was shaken at 55°C for 60 minutes. Next, DNA was obtained from 400 µL of the solution using Maxwell (registered trademark) RSC Cultured Cells DNA Kit (Promega).

As shown in Figs. 24 and 25, for Campylobacter jejuni, all the mixed bacteria had a good bacterial elimination ability equivalent to that of feces F.

### (Example 16)

As shown in the upper part of Fig. 26, Clostridium difficile (St. 630) was administered to germ-free mice, and one week later, mixed F37mix, K68mix, I42mix, K47mix, or feces F was administered. Note that K47mix denotes 47 strains isolated from the fecal sample derived from #K, and is duplicated with the 68 bacterial strains (K1 to K46 shown in Tables 1 and 2) except for 1 bacterial strain.

The bacterial solution of C. difficle was subjected to spore formation, adjusted to around 1×10^5 cells, and administered into the stomachs of the mice using a sonde. The spore formation was performed by culturing in Clospore medium for 8 days in an anaerobic chamber at 37°C and washing the medium with PBS, followed by treatment with sonication, addition of Lysoizyme and trypsin, and treatment at 45°C for 6 hours and then at 70°C for 10 minutes.

For C. difficle, qPCR was used to quantify the bacterial amount. As the primers for qPCR, the primer set shown in SEQ ID NOs: 224 and 225 was used.

As a result, as shown in the lower part of Fig. 26, a high elimination ability was observed in K68mix and K47mix for C. difficile.

### [Industrial Applicability]

As described above, by suppressing the colonization of drug-resistant bacteria and pro-inflammatory bacteria in the intestinal tract, the present invention makes it possible to treat, ameliorate, or prevent diseases caused by these bacteria. Therefore, the present invention is extremely useful in the development, treatment, amelioration, prevention, of pharmaceutical products relating to infectious diseases caused by drug-resistant bacteria or pro-inflammatory bacteria.

## Claims

1. An antibacterial composition against drug-resistant bacteria or pro-inflammatory bacteria, said composition comprising as the only active ingredients
(1) an intestinal bacterium having a DNA composed of a base sequence of SEQ ID NO: 105 or a base sequence having at least 90% identity with the base sequence,
(2) an intestinal bacterium having a DNA composed of a base sequence of SEQ ID NO: 69 or a base sequence having at least 90% identity with the base sequence,
(3) an intestinal bacterium having a DNA composed of a base sequence of SEQ ID NO: 80 or a base sequence having at least 90% identity with the base sequence,
(4) an intestinal bacterium having a DNA composed of a base sequence of SEQ ID NO: 85 or a base sequence having at least 90% identity with the base sequence,
(5) an intestinal bacterium having a DNA composed of a base sequence of SEQ ID NO: 86 or a base sequence having at least 90% identity with the base sequence,
(6) an intestinal bacterium having a DNA composed of a base sequence of SEQ ID NO: 87 or a base sequence having at least 90% identity with the base sequence,
(7) an intestinal bacterium having a DNA composed of a base sequence of SEQ ID NO: 88 or a base sequence having at least 90% identity with the base sequence,
(8) an intestinal bacterium having a DNA composed of a base sequence of SEQ ID NO: 89 or a base sequence having at least 90% identity with the base sequence,
(9) an intestinal bacterium having a DNA composed of a base sequence of SEQ ID NO: 90 or a base sequence having at least 90% identity with the base sequence,
(10) an intestinal bacterium having a DNA composed of a base sequence of SEQ ID NO: 91 or a base sequence having at least 90% identity with the base sequence,
(11) an intestinal bacterium having a DNA composed of a base sequence of SEQ ID NO: 92 or a base sequence having at least 90% identity with the base sequence,
(12) an intestinal bacterium having a DNA composed of a base sequence of SEQ ID NO: 94 or a base sequence having at least 90% identity with the base sequence,
(13) an intestinal bacterium having a DNA composed of a base sequence of SEQ ID NO: 96 or a base sequence having at least 90% identity with the base sequence,
(14) an intestinal bacterium having a DNA composed of a base sequence of SEQ ID NO: 98 or a base sequence having at least 90% identity with the base sequence,
(15) an intestinal bacterium having a DNA composed of a base sequence of SEQ ID NO: 99 or a base sequence having at least 90% identity with the base sequence,
(16) an intestinal bacterium having a DNA composed of a base sequence of SEQ ID NO: 100 or a base sequence having at least 90% identity with the base sequence,
(17) an intestinal bacterium having a DNA composed of a base sequence of SEQ ID NO: 101 or a base sequence having at least 90% identity with the base sequence, and
(18) an intestinal bacterium having a DNA composed of a base sequence of SEQ ID NO: 103 or a base sequence having at least 90% identity with the base sequence.

2. The antibacterial composition of claim 1, which is a pharmaceutical composition.

3. The antibacterial composition of claim 1 or 2 for use in treating, ameliorating, or preventing an infectious disease or an inflammatory disease.

4. The antibacterial composition according to claim 1, wherein the drug-resistant bacteria or pro-inflammatory bacteria is Clostridium difficile, Campylobacter jejuni or adherent-invasive Escherichia coli (AIEC).

5. The antibacterial composition according to claim 1, wherein the drug-resistant bacteria or pro-inflammatory bacteria is Clostridioides difficile (Prevot) Lawson et al. (ATCC 43255, bacterial strain designation: VPI 10463), Clostridioides difficile (Prevot) Lawson et al. (ATCC BAA-1382, bacterial strain designation: 630), Campylobacter jejuni 81-176 (ATCC BAA2151) or AIEC LF82.

## Patentansprüche

1. Antibakterielle Zusammensetzung gegen arzneimittelresistente Bakterien oder proinflammatorische Bakterien, wobei die Zusammensetzung als einzige Wirkstoffe umfasst:
(1) ein Darmbakterium mit einer DNA, die aus einer Basensequenz von SEQ ID NO: 105 oder einer Basensequenz mit mindestens 90% Identität mit der Basensequenz besteht,
(2) ein Darmbakterium mit einer DNA, die aus einer Basensequenz von SEQ ID NO: 69 oder einer Basensequenz mit mindestens 90% Identität mit der Basensequenz besteht,
(3) ein Darmbakterium mit einer DNA, die aus einer Basensequenz von SEQ ID NO: 80 oder einer Basensequenz mit mindestens 90% Identität mit der Basensequenz besteht,
(4) ein Darmbakterium mit einer DNA, die aus einer Basensequenz von SEQ ID NO: 85 oder einer Basensequenz mit mindestens 90% Identität mit der Basensequenz besteht,
(5) ein Darmbakterium mit einer DNA, die aus einer Basensequenz von SEQ ID NO: 86 oder einer Basensequenz mit mindestens 90% Identität mit der Basensequenz besteht,
(6) ein Darmbakterium mit einer DNA, die aus einer Basensequenz von SEQ ID NO: 87 oder einer Basensequenz mit mindestens 90% Identität mit der Basensequenz besteht,
(7) ein Darmbakterium mit einer DNA, die aus einer Basensequenz von SEQ ID NO: 88 oder einer Basensequenz mit mindestens 90% Identität mit der Basensequenz besteht,
(8) ein Darmbakterium mit einer DNA, die aus einer Basensequenz von SEQ ID NO: 89 oder einer Basensequenz mit mindestens 90% Identität mit der Basensequenz besteht,
(9) ein Darmbakterium mit einer DNA, die aus einer Basensequenz von SEQ ID NO: 90 oder einer Basensequenz mit mindestens 90% Identität mit der Basensequenz besteht,
(10) ein Darmbakterium mit einer DNA, die aus einer Basensequenz von SEQ ID NO: 91 oder einer Basensequenz mit mindestens 90% Identität mit der Basensequenz besteht,
(11) ein Darmbakterium mit einer DNA, die aus einer Basensequenz von SEQ ID NO: 92 oder einer Basensequenz mit mindestens 90% Identität mit der Basensequenz besteht,
(12) ein Darmbakterium mit einer DNA, die aus einer Basensequenz von SEQ ID NO: 94 oder einer Basensequenz mit mindestens 90% Identität mit der Basensequenz besteht,
(13) ein Darmbakterium mit einer DNA, die aus einer Basensequenz von SEQ ID NO: 96 oder einer Basensequenz mit mindestens 90% Identität mit der Basensequenz besteht,
(14) ein Darmbakterium mit einer DNA, die aus einer Basensequenz von SEQ ID NO: 98 oder einer Basensequenz mit mindestens 90% Identität mit der Basensequenz besteht,
(15) ein Darmbakterium mit einer DNA, die aus einer Basensequenz von SEQ ID NO: 99 oder einer Basensequenz mit mindestens 90% Identität mit der Basensequenz besteht,
(16) ein Darmbakterium mit einer DNA, die aus einer Basensequenz von SEQ ID NO: 100 oder einer Basensequenz mit mindestens 90% Identität mit der Basensequenz besteht,
(17) ein Darmbakterium mit einer DNA, die aus einer Basensequenz von SEQ ID NO: 101 oder einer Basensequenz mit mindestens 90% Identität mit der Basensequenz besteht, und
(18) ein Darmbakterium mit einer DNA, die aus einer Basensequenz von SEQ ID NO: 103 oder einer Basensequenz mit mindestens 90% Identität mit der Basensequenz besteht.

2. Antibakterielle Zusammensetzung nach Anspruch 1, die eine pharmazeutische Zusammensetzung ist.

3. Antibakterielle Zusammensetzung nach Anspruch 1 oder 2 zur Verwendung bei der Behandlung, Linderung oder Vorbeugung einer Infektionskrankheit oder einer Entzündungskrankheit.

4. Antibakterielle Zusammensetzung nach Anspruch 1, wobei das arzneimittelresistente Bakterium oder das proinflammatorische Bakterium Clostridium difficile, Campylobacter jejuni oder adhärent-invasive Escherichia coli (AIEC) ist.

5. Antibakterielle Zusammensetzung nach Anspruch 1, wobei das arzneimittelresistente Bakterium oder das proinflammatorische Bakterium Clostridioides difficile (Prevot) Lawson et al. (ATCC 43255, Bakterienstammbezeichnung: VPI 10463), Clostridioides difficile (Prevot) Lawson et al. (ATCC BAA-1382, Bakterienstammbezeichnung: 630), Campylobacter jejuni 81-176 (ATCC BAA2151) oder AIEC LF82 ist.

## Revendications

1. Composition antibactérienne contre des bactéries résistantes aux médicaments ou des bactéries pro-inflammatoires, ladite composition comprenant, en tant qu'ingrédients actifs uniques,
(1) une bactérie intestinale ayant un ADN composé d'une séquence de base de SEQ ID n° : 105 ou d'une séquence de base ayant au moins 90 % d'identité avec la séquence de base,
(2) une bactérie intestinale ayant un ADN composé d'une séquence de base de SEQ ID n° : 69 ou d'une séquence de base ayant au moins 90 % d'identité avec la séquence de base,
(3) une bactérie intestinale ayant un ADN composé d'une séquence de base de SEQ ID n° : 80 ou d'une séquence de base ayant au moins 90 % d'identité avec la séquence de base,
(4) une bactérie intestinale ayant un ADN composé d'une séquence de base de SEQ ID n° : 85 ou d'une séquence de base ayant au moins 90 % d'identité avec la séquence de base,
(5) une bactérie intestinale ayant un ADN composé d'une séquence de base de SEQ ID n° : 86 ou d'une séquence de base ayant au moins 90 % d'identité avec la séquence de base,
(6) une bactérie intestinale ayant un ADN composé d'une séquence de base de SEQ ID n° : 87 ou d'une séquence de base ayant au moins 90 % d'identité avec la séquence de base,
(7) une bactérie intestinale ayant un ADN composé d'une séquence de base de SEQ ID n° : 88 ou d'une séquence de base ayant au moins 90 % d'identité avec la séquence de base,
(8) une bactérie intestinale ayant un ADN composé d'une séquence de base de SEQ ID n° : 89 ou d'une séquence de base ayant au moins 90 % d'identité avec la séquence de base,
(9) une bactérie intestinale ayant un ADN composé d'une séquence de base de SEQ ID n° : 90 ou d'une séquence de base ayant au moins 90 % d'identité avec la séquence de base,
(10) une bactérie intestinale ayant un ADN composé d'une séquence de base de SEQ ID n° : 91 ou d'une séquence de base ayant au moins 90 % d'identité avec la séquence de base,
(11) une bactérie intestinale ayant un ADN composé d'une séquence de base de SEQ ID n° : 92 ou d'une séquence de base ayant au moins 90 % d'identité avec la séquence de base,
(12) une bactérie intestinale ayant un ADN composé d'une séquence de base de SEQ ID n° : 94 ou d'une séquence de base ayant au moins 90 % d'identité avec la séquence de base,
(13) une bactérie intestinale ayant un ADN composé d'une séquence de base de SEQ ID n° : 96 ou d'une séquence de base ayant au moins 90 % d'identité avec la séquence de base,
(14) une bactérie intestinale ayant un ADN composé d'une séquence de base de SEQ ID n° : 98 ou d'une séquence de base ayant au moins 90 % d'identité avec la séquence de base,
(15) une bactérie intestinale ayant un ADN composé d'une séquence de base de SEQ ID n° : 99 ou d'une séquence de base ayant au moins 90 % d'identité avec la séquence de base,
(16) une bactérie intestinale ayant un ADN composé d'une séquence de base de SEQ ID n° : 100 ou d'une séquence de base ayant au moins 90 % d'identité avec la séquence de base,
(17) une bactérie intestinale ayant un ADN composé d'une séquence de base de SEQ ID n° : 101 ou d'une séquence de base ayant au moins 90 % d'identité avec la séquence de base, et
(18) une bactérie intestinale ayant un ADN composé d'une séquence de base de SEQ ID n° : 103 ou d'une séquence de base ayant au moins 90 % d'identité avec la séquence de base.

2. Composition antibactérienne de la revendication 1, qui est une composition pharmaceutique.

3. Composition antibactérienne de la revendication 1 ou 2, pour utilisation dans le traitement, l'amélioration, ou la prévention d'une maladie infectieuse ou d'une maladie inflammatoire.

4. Composition antibactérienne selon la revendication 1, dans laquelle les bactéries résistantes aux médicaments ou bactéries pro-inflammatoires sont Clostridium difficile, Campylobacter jejuni, ou Escherichia coli adhérentes-invasives (AIEC).

5. Composition antibactérienne selon la revendication 1, dans laquelle les bactérie résistantes aux médicaments ou bactéries pro-inflammatoires sont Clostridioides difficile (Prévot) Lawson et al. (ATCC 43255, identification de souche bactérienne : VPI 10463), Clostridioides difficile (Prévot) Lawson et al. (ATCC BAA-1382, identification de souche bactérienne : 630), Campylobacter jejuni 81-176 (ATCC BAA2151) ou AIEC LF82.
